(19) Europäisches Patentamt
European Patent Office
Office européen des brevets

(11) **EP 2 167 009 B1**

(12) **EUROPÄISCHE PATENTSCHRIFT**

(45) Veröffentlichungstag und Bekanntmachung des Hinweises auf die Patenterteilung:
**25.01.2012 Patentblatt 2012/04**

(21) Anmeldenummer: **08759124.4**

(22) Anmeldetag: **09.06.2008**

(51) Int Cl.:
*A61H 33/06* (2006.01)          *A61N 5/06* (2006.01)
*F24J 2/10* (2006.01)          *F24J 2/16* (2006.01)
*F24J 2/18* (2006.01)          *G02B 17/00* (2006.01)

(86) Internationale Anmeldenummer:
**PCT/EP2008/004596**

(87) Internationale Veröffentlichungsnummer:
**WO 2008/151768 (18.12.2008 Gazette 2008/51)**

(54) **SONNENLICHTKAMMER, INSBESONDERE SONNENLICHTSAUNA ODER GEWÄCHSHAUS**

SUNLIGHT CHAMBER, PARTICULARLY SUNLIGHT SAUNA OR GREENHOUSE

CHAMBRE SOLAIRE, EN PARTICULIER SAUNA SOLAIRE OU SERRE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **11.06.2007 DE 102007027402**

(43) Veröffentlichungstag der Anmeldung:
**31.03.2010 Patentblatt 2010/13**

(73) Patentinhaber: **Abmayr, Bernhard**
**91077 Dormitz (DE)**

(72) Erfinder: **Abmayr, Bernhard**
**91077 Dormitz (DE)**

(74) Vertreter: **Schlögl, Markus et al**
**Meissner, Bolte & Partner GbR**
**Bankgasse 3**
**90402 Nürnberg (DE)**

(56) Entgegenhaltungen:
WO-A-93/25174          WO-A1-91/03682
DE-A1- 2 711 261          DE-A1- 4 242 258
DE-U1- 20 014 497          US-A- 4 003 638
US-A- 4 715 358          US-A- 4 974 922
US-A1- 2001 011 551

**Beschreibung**

Die Erfindung betrifft eine Anordnung umfassend eine Sonnenlichtsauna oder ein Gewächshaus.

Neben finnischen Saunen und Dampfsaunen sind Infrarot-Saunen, d.h. Saunakabinen mit Infrarot-Lampen, bekannt. Aus DE 19942632 A1 ist beispielsweise eine Infrarot-Sauna mit unisolierten Wänden, die mit Strahlungsreflektoren bestückt sind, bekannt.

Aus US 1772219 und FR 2785792 sind Saunen bekannt, die ausschließlich oder teilweise von der Sonne aufgeheizt werden. In DE 42 42 258 A1 ist eine Saunakabine mit einem Lichtgeber offenbart.

Aus US 4 974 922 ist eine hexagonale Kammer, in der man ein Lichtbad nehmen kann, bekannt. WO 93/25174 A beschreibt eine Behandlungs- bzw. Therapiekabine für energetische, kosmetische und medizinische Zwecke. In DE 200 14 497 U1 ist eine der Entspannung, dem Relaxen oder der Meditation dienende Pyramide offenbart.

Aus DE 199 37 448 A1 sind Trichterelemente mit verspiegelten Seitenwänden bekannt, die in der Lage sind, Licht zu fokussieren und damit auf einen kleinen Bereich zu konzentrieren.

Aus US 2001/0011551 A1 ist eine photovoltaische Vorrichtung mit einem optischen Konzentrator bekannt. US 4 715 358 beschreibt die automatische Steuerung einfallender Sonnenstrahlung. In US 4 003 638 ist eine Vorrichtung zur Sammlung und Konzentrierung von Sonnenenergie offenbart.

Des Weiteren sind Kabinen oder Räume, in denen die gesundheitsfördernde Wirkung der Sonnenstrahlung ausgenutzt wird, aus US 1772219 und EP 0114977 B1 bekannt.

Auch in Gewächshäusern wird die Wirkung der Sonnenstrahlung in geschützten Räumen ausgenutzt.

Diese die Sonnenstrahlung nutzenden Saunen bzw. Kabinen haben den Nachteil, dass die Sonnenstrahlung nur sehr ineffizient genutzt wird oder wegen der fehlenden Konzentrierung der Sonnenstrahlung ein Betrieb bei kühler Witterung eine Zusatzheizung erfordert.

Der vorliegenden Erfindung liegt die Aufgabe zugrunde, eine verbesserte Anordnung umfassend eine Sonnenlichtsauna oder ein Gewächshaus anzugeben. Die Sonnenstrahlung soll hierbei einerseits möglichst effizient genutzt werden, vorzugsweise soll sie verstärkt bzw. konzentriert werden. Andererseits soll die Strahlungsintensität bei Bedarf auch begrenzbar oder reduzierbar sein.

Diese Aufgabe wird gemäß der Erfindung durch eine Anordnung umfassend eine Sonnenlichtsauna oder ein Gewächshaus mit den Merkmalen nach Anspruch 1 gelöst. Vorteilhafte Ausgestaltungen und Weiterbildungen sind in den von Anspruch 1 abhängigen Ansprüchen angegeben.

Anspruch 1 sieht eine Anordnung umfassend eine Sonnenlichtsauna oder ein Gewächshaus (im Folgenden wird unter dem Begriff Sonnenlichtkammer eine Sonnenlichtsauna oder ein Gewächshaus verstanden) vor. Diese Sonnenlichtsauna bzw. das Gewächshaus weist einen Innenraum umschließende Wand- und/oder Deckenflächen und einen Boden auf. Mindestens eine erste der Wand- und/oder Deckenflächen, insbesondere eine der Sonne abgewandte Wand- und/oder Deckenfläche, und/oder der Boden ist zum Innenraum hin zumindest überwiegend gerichtet und/oder diffus reflektierend für sichtbares Licht und/oder infrarote Strahlung und/oder UV-Strahlung ausgebildet, insbesondere durch eine weiße oder helle oder spiegelnde Oberfläche. Mindestens eine zweite der Wand- und/oder Deckenflächen, insbesondere eine der Sonne zugewandte Wand- und/oder Deckenfläche, ist zumindest abschnittsweise, insbesondere auch zumindest überwiegend, transparent für sichtbares Licht und/oder infrarote Strahlung und/oder UV-Strahlung ausgebildet. An und/oder in der oder den zweiten Wand- und/oder Deckenflächen sind ein oder mehrere Lichttrichter derart angeordnet oder ausgebildet, dass eine engste Stelle des Lichttrichters dem Innenraum zugewandt ist. Dies bedeutet, dass entsprechend einfallende Sonnenstrahlen durch die engsten Stellen der Lichttrichter in den Innenraum eindringen können. Die Randflächen der Lichttrichter sind von gerichtet reflektierenden, insbesondere als Spiegelflächen ausgebildeten Seitenflächen zueinander benachbarter Spiegelkörper gebildet. Die Spiegelkörper weisen dem Innenraum zugewandte Basisflächen auf, zwischen denen die engsten Stellen der Lichttrichter ausgebildet sind. Die Basisflächen sind zum Innenraum hin zumindest überwiegend gerichtet und/oder diffus reflektierend für sichtbares Licht und/oder infrarote Strahlung und/oder UV-Strahlung ausgebildet.

Die Anordnung gemäß der Erfindung umfasst ferner mindestens eine Vorrichtung zur Begrenzung oder Reduzierung der Strahlungsintensität im Innenraum der Sonnenlichtsauna bzw. des Gewächshauses.

Die Vorteile dieser Anordnung gemäß der Erfindung liegen insbesondere darin, die natürliche Sonnenstrahlung einzufangen und zu konzentrieren. Hinsichtlich der Sauna erreicht man damit eine Kombination der gesundheitsfördernden Wirkung der Sonnenstrahlung und der inneren Reinigung und Entschlackung durch das Schwitzen. Die Konzentration bzw. Verstärkung der Strahlung ermöglicht darüber hinaus den Betrieb auch zu kühlen Jahreszeiten ohne Zusatzheizung.

Es sei darauf hingewiesen, dass der bzw. die Lichttrichter (bzw. allgemein eine Konzentrieranordnung) zwar das Sonnenlicht bündeln, so dass das Licht nicht über eine große Fläche, sondern in konzentrierter bzw. gebündelter Form, beispielsweise über einen schmalen Spalt, in das Innere der Kammer eingeleitet werden kann. Die Erhöhung der Strahlungsintensität im Inneren der Sonnenlichtkammer erfolgt jedoch nicht aufgrund dieser Lichtbündelung durch die Lichttrichter bzw. die Konzentrieranordnung selbst, sondern durch die Verspiegelung und die damit verbundene Lichtreflexion im Innenraum der Sonnenlichtkammer. Die Lichttrichter bzw. die Konzentrieranordnung dienen nur dazu, möglichst viel Sonnenlicht durch den Spalt in das Innere der Sonnenlichtkammer zu lenken. Hinter dem Spalt im Inneren der Kammer fächert sich das Licht sofort wieder auf, die Lichtkonzentrierung in der Sonnenlichtkammer wird nur durch die weitgehende Verspiegelung der Innenflächen der Kammer erreicht. Die Funktion des bzw. der Lichttrichter bzw. der Konzentrieranordnung liegt somit darin, die für den Lichteintritt erforderliche Fläche deutlich zu verringern und somit eine teilweise Verspiegelung der Innenseite der Lichteintrittsseite der Sonnenlichtkammer zu ermöglichen, wodurch der Lichtaustritt erschwert und die Lichtkonzentration im Inneren der Sonnenlichtkammer erhöht wird.

Das grundlegend Neue an der Sonnenlichtkammer gemäß der Erfindung in der Ausbildung als Sauna ist, dass der menschliche Körper nicht primär durch die künstlich oder natürlich aufgeheizte, warme oder heiße Umgebungsluft einer Sauna oder durch künstliche Infrarotstrahlung aufgeheizt wird, sondern direkt durch die natürliche Strahlung der Sonne. Mit der erfindungsgemäßen Sonnenlichtsauna lässt sich eine Verstärkung der Sonnenlichtstrahlung erreichen, was wiederum einen genügend großen Wärmeeintrag in den Körper auch bei kalten Umgebungsbedingungen ermöglicht. Die Sonnenlichtsauna ist somit hinsichtlich Einsatzort, Wetter und Jahreszeit flexibel einsetzbar. Ein weiterer Vorteil ist, dass sich in der Sonnenlichtsauna gemäß der Erfindung eine im Vergleich zum Stand der Technik höhere Strahlungsdichte erreichen lässt. Des Weiteren wird im Vergleich zum normalen Sonnenbad eine größere Oberfläche des Körpers bestrahlt, da die Strahlung aufgrund der reflektierenden Wand- bzw. Deckenflächen von allen Seiten auf den Körper trifft.

Da der sichtbare Anteil der Sonnenstrahlung viel tiefer in die Haut eindringt als fernes Infrarot, hat die Sonnenlichtsauna im Vergleich zu Infrarot-Saunen und zu normalen Saunen mit heißer Luft den Vorteil, dass primär nicht nur die nicht durchblutete Epidermis erwärmt wird, sondern ein viel größeres Hautvolumen und damit auch Hautschichten, die durchblutet sind. Dies vermeidet lokale Überhitzungen und beschleunigt den Wärmetransport ins Körperinnere durch das Blut.

Sollte die Intensität der Sonnenstrahlung im Winter bei kleineren Sonnenlichtsaunen nicht für einen Saunaeffekt ausreichen, ist es immerhin möglich, bei derartigen Sonnenlichtsaunen nach der Erfindung dem Körper durch das gesammelte Sonnenlicht ein intensives Sonnenbad und damit eine Lichttherapie zu gönnen.

Dieses für Sonnenlichtsaunen vorteilhafte Prinzip lässt sich auch vorteilhaft für neuartige Gewächshäuser verwenden, insbesondere für Gewächshäuser, die auch im Winter eine möglichst intensive Sonnenstrahlung für Pflanzen bereitstellen sollen.

Gemäß einer Weiterbildung umfasst die mindestens eine Vorrichtung zur Begrenzung oder Reduzierung der Strahlungsintensität im Innenraum eine einstellbare Beschattungsvorrichtung außerhalb der Sonnenlichtkammer und/oder die Ausbildung der zweiten Wand- und/oder Deckenflächen aus Glas- und/oder Plexiglasscheiben mit, vorzugsweise durch Anlegen einer elektrischen Spannung, veränderbarem Transmissionsvermögen. Auch kann die Vorrichtung zur Begrenzung oder Reduzierung der Strahlungsintensität die steuerbare Ein- und/oder Ausfahrung von Solarzellen, insbesondere Konzentratorzellen, und/oder das Einstellen strahlungsabsorbierender Gegenstände, vorzugsweise dunkler Steine, in den Innenraum umfassen. Vorgesehen werden kann auch das Öffnen oder Entfernen einer spiegelnden Abdeckung vor strahlungsabsorbierenden Gegenständen im Innenraum und/oder die Ausleitung von Strahlung, vorzugsweise mittels in ersten Wand- und/oder Deckenflächen oder im Boden angeordneter Lichtleiter.

Gemäß einer Weiterbildung sind die Lichttrichter derart ausgebildet und angeordnet, dass auftreffende Sonnenstrahlung in den Innenraum gelenkt wird, ein Austreten der Strahlung aus dem Innenraum jedoch zumindest teilweise verhindert ist, so dass der Innenraum einen Lichtkäfig bildet.

In einer Ausbildung weisen die Spiegelkörper der Lichttrichter jeweils in einem Querschnitt vertikal zur Basisfläche zumindest im Wesentlichen eine Dreiecksform auf, insbesondere die Form eines gleichschenkligen Dreiecks, bei dem die Seitenflächen des Spiegelkörpers die Schenkel und die Basisfläche die Basis bilden. Der Lichttrichter kann dann im vorgenannten Querschnitt die Form eines Dreiecks, insbesondere eines gleichschenkligen Dreiecks, mit stumpfer Spitze an der engsten Stelle des Lichttrichters aufweisen.

Im vorgenannten Querschnitt grenzen bei einer Weiterbildung die Basisflächen der Spiegelkörper und die engsten Stellen der Lichttrichter aneinander, wobei die Basisflächen jeweils eine vorgegebene Basisbreite und die engsten Stellen jeweils eine vorgegebene Trichterbreite aufweisen, und wobei der Quotient Basisbreite durch Trichterbreite im Bereich von 0 bis 100, insbesondere von 1 bis 100, vorzugsweise von 1 bis 25, liegt.

Gemäß einer Ausführungsvariante ist vorgesehen, dass im vorgenannten Querschnitt der Basiswinkel des Spiegelkörpers zwischen Basisfläche und Seitenflächen im Bereich von 50° bis 89°, insbesondere im Bereich von 60° bis 88°, liegt.

Auch kann vorgesehen sein, dass der Quotient Basisbreite durch Trichterbreite in Abhängigkeit von der gewählten Größe des Basiswinkels bestimmt ist, wobei insbesondere die Trichterbreite vorgegeben ist und die Basisbreite derart bestimmt ist, dass auch am oder nahe dem äußeren Rand des Lichttrichters auftreffende Sonnenstrahlung noch durch die engste Stelle in den Innenraum eindringt, wobei vorzugsweise bei einem Basiswinkel von etwa 60° der Quotient Basisbreite durch Trichterbreite etwa 1,0 beträgt, bei einem Basiswinkel von etwa 65° etwa 1,3, bei einem Basiswinkel von etwa 70° etwa 1,9, bei einem Basiswinkel von etwa 75° etwa 2,8, bei einem Basiswinkel von etwa 80° etwa 4,8, bei einem Basiswinkel von etwa 85° etwa 10,4 und bei einem Basiswinkel von etwa 87,5° etwa 21,8.

In einer Ausführungsvariante sind der oder die Lichttrichter entweder als eindimensionale Lichttrichter mit zwei einander gegenüberliegenden Randflächen und einer schlitzförmigen engsten Stelle oder als zweidimensionale Lichttrichter mit vier jeweils paarweise einander gegenüberliegenden Randflächen und einer zumindest im Wesentlichen zweidimensionalen, insbesondere rechteckigen oder quadratischen engsten Stelle ausgebildet.

Auch kann die zweite Wand- und/oder Deckenfläche oder können die zweiten Wand- und/oder Deckenflächen eine oder mehrere, insbesondere entspiegelte, zumindest teilweise lichtdurchlässige und/oder transparente Scheiben und/oder Folien umfassen, insbesondere aus Glas und/oder Plexiglas. In Betracht kommt auch jedes andere zumindest teilweise lichtdurchlässige und/oder transparente Material. Dadurch wird ein hohes Transmissionsvermögen sichergestellt.

Gemäß einer zweckmäßigen Ausführungsvariante ist bzw. sind der oder die Lichttrichter im Innenraum, insbesondere unterhalb der zweiten Wand-und/oder Deckenfläche oder der zweiten Wand- und/oder Deckenflächen bzw. unterhalb der Glas- und/oder Plexiglasscheibe, angeordnet. Dadurch wird ein Verdrecken der Lichttrichter verhindert, die darüber liegende zweite Wand- und/oder Deckenfläche bzw. Glas- und/oder Plexiglasscheibe ist nicht derart anfällig für Verschmutzung, sie ist einfach zu reinigen bzw. reinigt sich gegebenenfalls sogar selbst.

Bei einer anderen Ausführungsvariante sind die Lichttrichter außerhalb des Innenraums angeordnet. Es ist auch vorstellbar, dass einige Lichttrichter innerhalb und einige Lichttrichter außerhalb des Innenraums angeordnet sind. Während im Innenraum die Verwendung vieler kleiner Lichttrichter nebeneinander sinnvoll ist, damit nicht zuviel Raum dafür benötigt wird, können auf der Außenseite auch wenige große Lichttrichter verwendet werden. Es ist sogar möglich, nur einen einzigen Lichttrichter zu verwenden, der lediglich aus den beiden Seitenflächen besteht. Als Spalt dient dann die ganze zweite Wand- und/oder Deckenfläche oder ein Teil davon. Die Seitenflächen des Lichttrichters können weit über die Seiten des Innenraums bzw. der Lichtkammer hinausragen. Dadurch ist es möglich, sehr viel mehr Licht einzufangen. Allerdings ist zu bedenken, dass die Höhe der Lichttrichter meist ein Vielfaches der Spaltbreite ist, beispielsweise das 12-fache. Demnach sind dieser Variante oft konstruktive Grenzen gesetzt. Zur Lösung solcher Probleme können als Seitenflächen Spiegel verwendet werden, die mit einem Klappmechanismus zusammenlegbar sind. Dann können sie bei Bedarf in ihre wahre Größe aufgeklappt werden und sind sonst zusammengelegt und damit gut geschützt. Neben der Vergrößerung der Lichteinfangfläche bietet die Anbringung der Lichttrichter außerhalb der Kammer bzw. des Innenraums noch weitere Vorteile: Die Spiegel beschlagen nicht so leicht und im Innenraum geht kein Platz durch die Anbringung der Lichttrichter verloren. Der Nachteil ist, dass die Spiegel schneller verschmutzen und der vollen Kraft der Witterung ausgesetzt sind (Wind, Regen, Schnee, Hagel). Deshalb dürfte diese Variante besonders für niederschlagsarme Regionen mit sauberer Luft geeignet sein.

Es ist auch möglich, dass die Lichttrichter in die zweite Wand- und/oder Deckenfläche zumindest teilweise integriert sind. Beispielsweise können die Lichttrichter in diesem Fall verhältnismäßig kleine Abmessungen von nur einigen Millimetern oder Zentimetern aufweisen. Diese Variante ist unter anderem leicht zu montieren.

Anstelle der Lichttrichter können auch Linsen oder Systeme aus Linsen und Spiegeln verwendet werden, um das Licht durch den engen Spalt zu lenken. Insbesondere eignen sich dazu zylindrische oder halbzylindrische Linsen, die das Licht durch einen transparenten Abschnitt, beispielsweise einen Spalt entsprechend dem Lichttrichterspalt, führen. Mit guten Linsen kann der Spalt viel enger ausgeführt werden als dies mit Lichttrichtern praktisch möglich ist.

Nach einer Weiterbildung ist bzw. sind der oder die Lichttrichter und/oder die zweite(n) Wand- und/oder Dekkenflächen(n) und/oder die gesamte Sonnenlichtkammer mit einer Einrichtung zur manuellen und/oder automatischen Sonnenstandsnachführung versehen, d.h. die jeweiligen Einrichtungen sind beweglich ausgebildet und mit einer entsprechenden Antriebs- und Steuereinrichtung versehen.

Eine weitere Ausführungsvariante der Erfindung sieht vor, dass eine Ausstattung der Sonnenlichtkammer, insbesondere Sitz- und/oder Liegeeinrichtungen, zumindest überwiegend gerichtet und/oder diffus reflektierend für sichtbares Licht und/oder infrarote Strahlung und/oder UV-Strahlung und/oder transparent hierfür ausgebildet ist.

Auch kann vorgesehen sein, dass zusätzliche Sonnenstrahlung, die nicht direkt auf die Sonnenlichtkammer auftrifft, in den Innenraum geleitet wird, insbesondere über externe Spiegel und/oder mittels Lichtleitern, insbesondere Glasfasern, über die vorzugsweise durch eine oder mehrere Sammellinsen gebündelte Sonnenstrahlung in den Innenraum leitbar ist und/oder über die mittels extern angeordneter, insbesondere zweidimensionaler Lichttrichter gebündeltes Licht von der engsten Stelle der Lichttrichter in den Innenraum leitbar ist.

Zusätzlich kann künstliche Strahlung im Innenraum erzeugt und/oder in den Innenraum geleitet werden, insbesondere über externe Spiegel und/oder mittels Lichtleitern, insbesondere Glasfasern.

Gemäß einer Ausbildung der Erfindung sind mindestens eine Tür und/oder mindestens eine Ent- und/oder Belüftung und/oder mindestens eine Leitungsdurchführung vorgesehen, wobei diese derart ausgebildet sind, dass zumindest weitestgehend keine Strahlung aus dem Innenraum heraus dringt. Eine zweckmäßige Variante sieht vor, mindestens eine Messeinrichtung zur Ermittlung der Strahlungsintensität im Innenraum und mindestens eine Anzeige für die ermittelten Messwerte im Innenraum und/oder außerhalb der Sonnenlichtkammer vorzusehen.

Gemäß einer Ausführungsvariante ist die Sonnenlichtkammer ausgebildet als freistehendes Bauwerk und/oder als Anbau an Gebäude und/oder als mobile Einrichtung, insbesondere als Aufbau auf einem Kraftfahrzeug oder Kraftfahrzeug-Anhänger und/oder als zusammenlegbarer und transportabler Bausatz für einen flexiblen Einsatz.

In einer speziellen Ausbildung ist die Sonnenlichtkammer in Pyramidenform ausgebildet, insbesondere mit einer quadratischen Grundfläche und/oder mit einem Quotienten Höhe durch Basisbreite von etwa 1,618 (Goldener Schnitt) und/oder mit einem Neigungswinkel der Pyramidenseiten von etwa 60° bis 85°, vorzugsweise von etwa 72° bis 73°. In oder an der Pyramide, insbesondere an der Pyramidenspitze, können ein oder mehrere Objekte zur Modifizierung des feinstofflichen Schwingungsspektrums, insbesondere Edelsteine und/oder Kristalle, vorgesehen sein, die insbesondere in einer speziellen Halterung angeordnet werden.

Eine Weiterbildung der Sonnenlichtkammer sieht ferner die Anordnung einer Blitzschutzeinrichtung vor.

In einer Ausbildung der Sonnenlichtkammer wird die Strahlung an bestimmten Stellen im Innenraum zusätzlich konzentriert, insbesondere durch eine entsprechende geometrische Konstruktion der, vorzugsweise verspiegelten, Wand- und/oder Deckenflächen und/oder des, vorzugsweise verspiegelten, Bodens und/oder durch Vorsehen von, vorzugsweise verspiegelten, Strahlungslenkungs-Einbauten im Innenraum. In einer Sonnenlichtsauna erfolgt die Konzentration der Strahlung insbesondere dort, wo sich die Menschen aufhalten. Die Konzentration der Strahlung kann erreicht werden durch eine intelligente Ausnutzung der Reflexionsgesetze für die Strahlung durch eine geeignete geometrische Konstruktion der Wand-, Decken- und Bodenflächen, insbesondere wenn diese verspiegelt sind, und/oder durch Verwendung zusätzlicher Einbauten im Innenraum zur Strahlungslenkung, insbesondere wenn diese verspiegelt sind.

Eine Weiterbildung der Erfindung sieht eine Konzentrieranordnung zur Konzentrierung bzw. Bündelung von Sonnenlicht vor, mittels der für die Einleitung in die Sonnenlichtkammer bestimmtes Sonnenlicht konzentrierbar ist. Diese Konzentrieranordnung kann zumindest teilweise ein Bestandteil der Sonnenlichtkammer sein, sie kann aber auch zumindest teilweise räumlich von der Sonnenlichtkammer getrennt vorgesehen sein. Bei einer räumlichen Trennung ist die Konzentrieranordnung jedoch lichttechnisch mit der Sonnenlichtkammer verbunden, insbesondere über Lichtleiter, vorzugsweise Glasfaser, und/oder einen oder mehrere Spiegel. Damit kann auch Sonnenstrahlung, die nicht direkt auf die Sonnenlichtkammer auftrifft, in die Sonnenlichtkammer geleitet werden. Eine externe Anordnung der Konzentrieranordnung außerhalb der Sonnenlichtkammer hat den Vorteil, dass nur die Konzentrieranordnung dem Sonnenstand nachzuführen ist, nicht jedoch die gesamte Kammer. Lässt sich die Konzentrieranordnung in einen Sammelteil zum Sammeln der Sonnenstrahlung und einen Konzentrierungteil zum Bündeln der Strahlung aufteilen, so ist es gegebenenfalls sogar ausreichend, nur den Sammelteil dem Sonnenstand nachzuführen.

Die konkreten Ausbildungsmöglichkeiten der Konzentrieranordnung sind vielfältig. Hinsichtlich der Konzentrieranordnung kommen verschiedenste Methoden zum Einfangen und Bündeln der Sonnenstrahlen in Betracht.

In einer Ausführungsvariante ist die Konzentrieranordnung räumlich vollständig von der Sonnenlichtkammer getrennt. Der Innenraum der Sonnenlichtkammer kann zumindest nahezu vollständig nach innen verspiegelt sein. Während bei der Anordnung von Konzentrieranordnungen direkt an der Sonnenlichtkammer dort in der Regel zumindest kleine transparente Abschnitte zur Einleitung des Lichts erforderlich sind, können bei einer vollständigen räumlichen Trennung jedoch alle Wände, Decken und der Boden vollständig oder zumindest fast vollständig (gegebenenfalls bis auf einen kleinen Einlass für einen Lichtleiter) verspiegelt sein, so dass es dann zumindest nahezu keine Spaltverluste mehr gibt.

Die Konzentrieranordnung kann gemäß einer Variante derart ausgebildet sein, dass sie Sonnenlicht in einer Einfallrichtung stärker hindurch lässt als in einer Ausfallrichtung. Hierzu kann die Konzentrieranordnung ein Material umfassen, das Sonnenlicht in einer Einfallrichtung stärker hindurch lässt als in einer Ausfallrichtung, insbesondere ein optisch anisotropes Material und/oder strahlungseinfangende und -leitende Polymere.

Gemäß einer anderen Variante kann die Konzentrieranordnung eine oder mehrere Linsen oder ein System aus Linsen und Spiegeln umfassen, mittels der oder denen gebündelte Sonnenstrahlung in die Sonnenlichtkammer leitbar ist, beispielsweise durch einen engen Spalt. Insbesondere eignen sich dazu zylindrische oder halbzylindrische Linsen, die das Licht durch einen transparenten Abschnitt, beispielsweise einen Spalt entsprechend dem bereits beschriebenen Lichttrichterspalt, führen. Mit guten Linsen kann der Spalt viel enger ausgeführt werden als dies mit Lichttrichtern praktisch möglich ist.

In einer Weiterbildung der allgemeinen erfindungsgemäßen Form der Sonnenlichtkammer weist zumindest eine Wand- und/oder Deckenfläche der Sonnenlichtkammer zumindest einen für sichtbares Licht und/oder infrarote Strahlung und/oder UV-Strahlung transparenten Abschnitt auf, durch den das von der Konzentrieranordnung konzentrierte Sonnenlicht in die Sonnenlichtkammer einleitbar ist.

Eine Ausführungsform sieht vor, dass die Konzentrieranordnung einen oder mehrere, an und/oder in der Sonnenlichtkammer und/oder räumlich getrennt von der Sonnenlichtkammer angeordnete Lichttrichter umfasst, mittels denen gebündelte Sonnenstrahlung von einer engsten Stelle der Lichttrichter in die Sonnenlichtsauna leitbar ist. Bei den Lichttrichtern kann es sich um eindimensionale Lichttrichter handeln, bei denen die Nachführung nur in einem Winkel nötig ist, d.h. nur Azimut oder Elevation. Es kann sich aber auch um einen zweidimensionalen Lichttrichter handeln, der für einen optimalen Betrieb eine Nachführung in beiden Winkeln, d.h. Azimut und Elevation, erfordert.

Eine Weiterbildung sieht wiederum vor, dass zusätzliche künstliche Strahlung in der Sonnenlichtkammer erzeugt und/oder in die Sonnenlichtsauna geleitet wird, insbesondere über externe Spiegel und/oder mittels Lichtleitern, insbesondere Glasfasern.

Die Erfindung wird nachstehend auch hinsichtlich weiterer Merkmale und Vorteile anhand der Beschreibung von Ausführungsbeispielen und unter Bezugnahme auf die beiliegenden, schematischen Zeichnungen näher erläutert. Dabei zeigen

FIG 1          in einer schematischen dreidimensionalen Darstellung ein Ausführungsbeispiel einer erfindungsgemäßen Anordnung,

FIG 2 und FIG 3        jeweils in einer schematischen Querschnittsdarstellung das Prinzip der gemäß der Erfindung einzusetzenden Lichttrichter, FIG 2 hinsichtlich des konstruktiven Aufbaus und FIG 3 hinsichtlich der Sonnenstrahlführung, und

FIG 4          eine spezielle Ausgestaltung der Anordnung nach FIG 1 in einer seitlichen Querschnittsdarstellung der Kammerspitze.

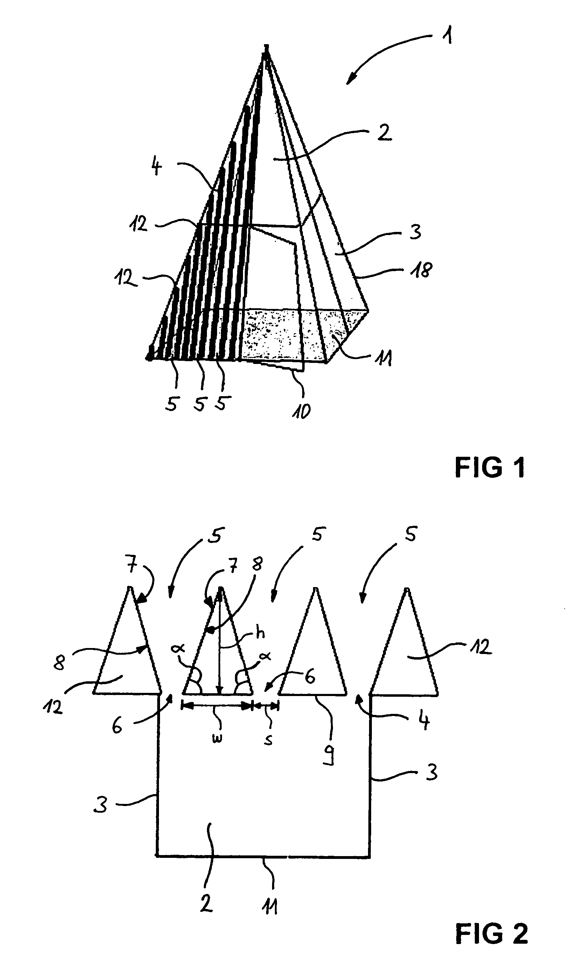

Einander entsprechende Komponenten und Teile sind in FIG 1 bis FIG 4 mit den gleichen Bezugszeichen bezeichnet.

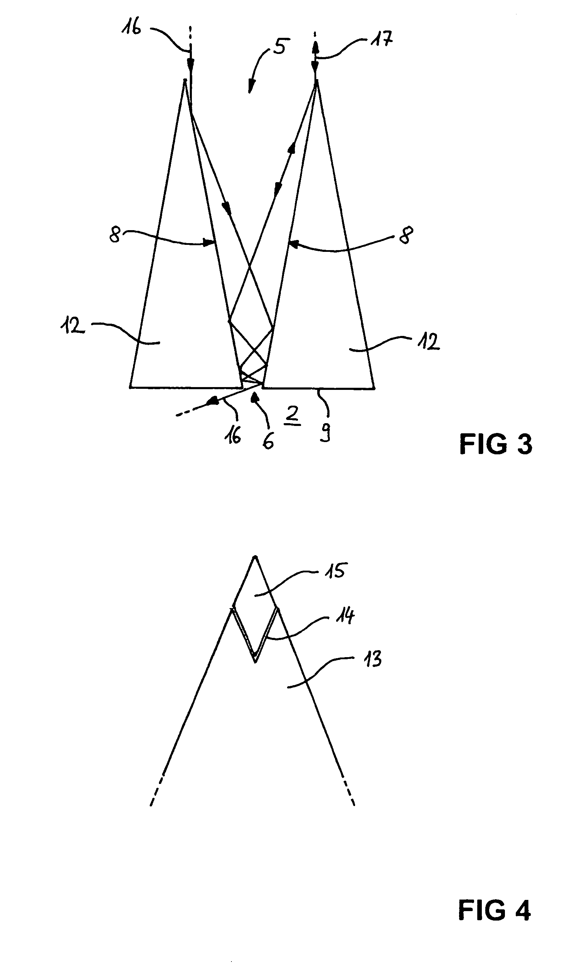

FIG 1 zeigt in einer schematischen dreidimensionalen Darstellung ein Ausführungsbeispiel einer erfindungsgemäßen Sonnenlichtkammer 1 (Sonnenlichtsauna oder Gewächshaus). Die Sonnenlichtkammer 1 weist eine Pyramidenform mit quadratischer Basisfläche auf. Diese Basisfläche bildet einen Boden 11 der Sonnenlichtkammer 1. Die vier Seitenflächen der Sonnenlichtkammer 1 sind von Wandflächen 3, 4 gebildet, die einen Innenraum 2 der Sonnenlichtkabine umschließen. Die Grundstruktur dieser Wandflächen 3, 4 und damit der Sonnenlichtkammer 1 insgesamt wird von einem metallenen Traggerüst 18 gebildet. Erste Wandflächen 3 sind der Sonne abgewandt, zweite Wandflächen 4 sind der Sonne zugewandt. Dementsprechend sind die ersten Wandflächen 3 und bevorzugt auch der Boden 11 zum Innenraum hin zumindest überwiegend gerichtet und/oder diffus reflektierend für sichtbares Licht und/oder infrarote Strahlung und/oder UV-Strahlung ausgebildet, beispielsweise durch Anordnung planer Spiegelelemente. Die zweiten Wandflächen 4 hingegen sind transparent für sichtbares Licht und/oder infrarote Strahlung und/oder UV-Strahlung ausgebildet. An ihnen sind Spiegelkörper 12 angeordnet, zwischen denen jeweils Lichttrichter 5 ausgebildet sind, die die Sonnenstrahlung gebündelt und damit konzentriert in den Innenraum 2 der Sonnenlichtkammer 1 leiten. Die Ausbildung, Anordnung und Funktion der Spiegelkörper 12 und der Lichttrichter 5 ist untenstehend anhand von FIG 2 und FIG 3 erläutert.

In FIG 1 ist noch eine Tür 11 der Sonnenlichtkammer 1 dargestellt. Diese liegt hier in einer zweiten Wandfläche 4 auf der sonnenzugewandten Seite der Sonnenlichtkammer 1, wobei jedoch eine Anordnung in einer ersten, der Sonne abgewandten Wandfläche 3 bevorzugt ist, da sich dann die sonnenzugewandten zweiten Flächen 4 vollständig mit Spiegelkörpern 12 und damit Lichttrichtern 5 ausstatten lassen.

Die Sonnenlichtkammer 1 nach FIG 1 ist insbesondere als Sonnenlichtsauna einsetzbar. Gegebenenfalls kann hierzu noch eine geeignete Ausstattung, beispielsweise Sitz- oder Liegeeinrichtungen mit reflektierenden Oberflächen, vorgesehen werden.

FIG 2 und FIG 3 zeigen in einer schematischen Querschnittsdarstellung das Prinzip der gemäß der Erfindung in der Sonnenlichtkammer 1 einzusetzenden Lichttrichter 5. Der konstruktive Aufbau ist in FIG 2 ersichtlich. Die sich ergebende Führung von Sonnenstrahlen 16, 17 ist beispielhaft in FIG 3 dargestellt.

FIG 2 zeigt einen von nach innen hin verspiegelten ersten Wandflächen 3 und einem Boden 11 umgrenzten Innenraum 2, der den Innenraum 2 einer kubischen Sonnenlichtkammer darstellt. Die Oberseite des Innenraums 2 wird von einer hinzugedachten zweiten Deckenfläche 4 begrenzt. An dieser Deckenfläche 4 sind Spiegelkörper 12 angeordnet, deren Querschnittsform ein gleichschenkliges Dreieck ist. Die Spiegelkörper 12 erstrecken sich selbstverständlich wie alle in FIG 2 dargestellten Objekte senkrecht zur Zeichenebene, d.h. alle Objekte sind räumlich ausgebildet. Jeder Spiegelkörper 12 weist eine dem Innenraum zugewandte und zu diesem hin verspiegelte Basisfläche 9 sowie zwei ebenfalls verspiegelte Seitenflächen 8 auf. Jeweils zwei einander gegenüberliegende Seitenflächen 8 benachbarter Spiegelkörper 12 bilden einen Lichttrichter 5 mit einer engsten Stelle 6 und mit von den Seitenflächen 8 gebildeten Randflächen 7. Die engsten Stellen 6 der Lichttrichter 5 sind dem Innenraum 2 zugewandt.

Die Basisfläche 9 des Spiegelkörpers 12 hat im dargestellten Querschnitt senkrecht zur Basisfläche eine Breite w, der Spiegelkörper 12 hat eine Höhe h über seiner Basisfläche 9. Die Basisfläche 9 schließt mit beiden Seitenflächen 8 den Winkel $\alpha$ ein. Ferner ist in FIG 2 die Breite s der engsten Stelle 6 des Lichttrichters 5 dargestellt.

FIG 3 zeigt beispielhaft den Strahlenverlauf für zwei nahe der Spitze der Spiegelkörper 12 auftreffende, senkrecht von oben einfallende Sonnenstrahlen 16, 17. Die Strahlen 16, 17 werden jeweils an den spiegelnden Seitenflächen 8 reflektiert und dadurch beide zunächst nach unten in den Lichttrichter 5 hinein, auf dessen engste Stelle 6 zu, geleitet. Der zunächst etwas weiter unten auftreffende Stahl 16 wird dabei durch die engste Stelle 6 des Lichttrichters 5 hindurch in den Innenraum 2 gelenkt. Strahl 16 ist hier (zumindest nahezu) der Grenzstrahl, der gerade noch in den Innenraum 2 gelangt. Alle senkrecht von oben einfallenden Strahlen, die unterhalb von Strahl 16 erstmals an den spiegelnden Seitenflächen 8 auftreffen, gelangen in den Innenraum 2. Alle Strahlen, die oberhalb von Strahl 16 auftreffen, werden durch den Lichttrichter 5 nach oben reflektiert und gelangen somit nicht in den Innenraum 2. Ein Beispiel ist der zunächst weiter oben als Strahl 16, d.h. näher an der Spitze des Spiegelkörpers 12 auftreffende Strahl 17. Dieser trifft kurz oberhalb der engsten Stelle 6 senkrecht auf die Seitenfläche 8 auf und wird somit in sich selbst zurückreflektiert und verlässt auf diese Weise den Lichttrichter 5 wieder in sich selbst senkrecht nach oben. Strahl 17 gelangt somit nicht in den Innenraum 2. Die Tatsache, dass Strahl 17 in sich selbst reflektiert wird, ist nur ein Spezialfall. In der Regel werden die nicht in den Innenraum 2 eintretenden Strahlen schräg nach oben reflektiert.

Da die Basisflächen 9 und die ersten Wandflächen 3 verspiegelt sind und die engsten Stellen 6 die einzigen, im Vergleich zur Basisfläche 9 äußerst engen Austrittsmöglichkeiten nach außen sind, ist ein Austritt eines einmal in den Innenraum 2 eingetretenen Strahls so erschwert, dass die meisten Strahlen zunächst vielfach im Innenraum 2 reflektiert werden, bevor sie diesen durch eine der engsten Stellen 6 wieder verlassen. Der Innenraum 2 bildet somit einen Lichtkäfig, in dem sich die Sonnenstrahlung konzentriert bzw. verstärkt.

FIG 4 zeigt in einer seitlichen Querschnittsdarstellung eine spezielle Ausgestaltung der Pyramidenspitze 13 der Sonnenlichtkammer 1 nach FIG 1. Dort ist eine spezielle Halterung für einen Edelstein 15 bzw. Kristall·15 ausgebildet.

Grundsätzlich sind sehr verschiedene Konstruktionen der Sonnenlichtkammer bzw. Sonnenlichtsauna möglich. Denkbar sind viele Formen von alleinstehenden Bauwerken, aber ebenso die Integration in bestehende Bauwerke, z.B. auf der Südseite einer Saunaanlage. Wesentlich ist bei jeder Konstruktion, darauf zu achten, dass zum einen genügend Sonnenstrahlung ins Innere gelangt, und dass zum anderen im Inneren möglichst wenig Verluste entstehen, beispielsweise durch Strahlung absorbierende Gegenstände und/oder durch ein die einzelnen Komponenten der Sonnenlichtkammer tragendes Gerüst. Der zweite Aspekt wird bei den Sonnenlichtkammern gemäß der Erfindung dadurch erreicht, dass die Wände, durch die keine Strahlung hereinkommt, d.h. die der Sonne abgewandt sind, aus Materialien mit möglichst hoher Reflexion der sichtbaren, infraroten und, bei Bedarf, auch der UV-Strahlung gebildet sind. Dabei spielt es keine Rolle, ob die Reflexion diffus (z.B. weiße Wand) oder gerichtet (z.B. Spiegel) ist. Dasselbe gilt für den Boden und die ganze Ausstattung sowie für alle anderen von innen sichtbaren Teile.

Kritisch in Bezug auf Verluste ist vor allem die Seite, durch die die Strahlung in den Innenraum eindringt. Denn an den Stellen, an denen Strahlung eintritt, kann sie auch wieder austreten. Dies wird gemäß der Erfindung durch eine spezielle Konstruktion, die Ausbildung von Lichttrichtern, weitgehend verhindert, wodurch sich eine nennenswerte Lichtanreicherung im Innenraum erzielen lässt.

Mit einer Anordnung von Lichttrichtern, wie sie schematisch in der vorstehend erläuterten FIG 2 gezeigt ist, lässt sich nahezu die gesamte auftreffende Sonnenstrahlung in den Innenraum leiten. Der Innenraum stellt somit einen Lichtkäfig dar. Sowohl die Innenwände des Lichtkäfigs, als auch die Außenseiten der im Querschnitt gleichschenkligen Spiegelelemente sind plane Spiegel mit hohem Reflexionsgrad, der für die nachfolgenden theoretischen Überlegungen auf 1,00 gesetzt wird.

Wenn die Sonnenstrahlung senkrecht von oben kommt, gelangt bei geeigneter Konstruktion die gesamte Strahlung teilweise direkt, teilweise durch mehrfache Reflexion an den Randflächen der Lichttrichter in den Lichtkäfig (Innenraum). Wenn nun ein Strahl nach ein- oder mehrfacher Reflexion im Käfig nach oben an die gedachte Deckenfläche des Lichtkäfigs gelangt, so trifft er entweder auf einen Spalt der Breite s (engste Stelle der Lichttrichter) und entweicht ins Freie, oder er trifft auf die verspiegelte Basisfläche des Spiegelkörpers (Breite w) und wird von dort in den Lichtkäfig zurück reflektiert (siehe FIG 2). Für die nachfolgenden, an FIG 2 orientierten Überlegungen wird vernachlässigt, dass bei genau senkrechtem Einfall der Anteil der Strahlung, der ohne Reflexion direkt durch den Spalt eindringt, durch den Boden sofort wieder senkrecht nach oben hinausreflektiert wird (dies lässt sich im übrigen durch einen schiefen Boden vermeiden).

Um eine Vorstellung von der möglichen Lichtanreicherung zu bekommen, wird angenommen, dass alle Begrenzungsflächen des Innenraums ideal verspiegelt sind (100% Reflexion), dass im Innenraum keinerlei Absorber (Einrichtung, Menschen, etc.) sind und dass die Strahlungsverteilung im Innenraum absolut isotrop ist. Wenn nun die Strahlung mit der Intensität $L_0$ eingeschaltet wird, wird sich die Strahlungsintensität $L_1$ im Innenraum solange (mit Lichtgeschwindigkeit) erhöhen, bis die durch die Spalte nach außen dringende Strahlung $L_2$ genauso groß wie $L_0$ ist, also $L_2 = L_0$. Alle Lichtstrahlen, die durch die Reflexionen im Innenraum nach oben laufen und dann an die gedachte Decke gelangen, treffen entweder auf einen Spalt der Breite s und gelangen damit ins Freie oder sie treffen auf die Basisfläche eines Spiegelkörpers mit der Breite w und werden wieder nach innen reflektiert. Das bedeutet, dass ein Anteil s/(s+w) dieser Strahlung nach außen gelangt und ein Anteil w/(s+w) im Innenraum verbleibt.

Damit ist

$$L_2 = L_1 * s/(s+w).$$

$$Da\ L_2 = L_0,$$

ist

$$L_1 = L_0 * (s+w)\ /\ s = L_0 * (1\ +\ w/s)$$

Damit ist die Strahlungsintensität im Innenraum um den Faktor w/s erhöht. Zusätzlich ist zu bedenken, dass diese Strahlungsintensität im Innenraum unter der Annahme der Isotropie in alle drei Raumrichtungen jeweils vor und zurück geht, während die einfallende Strahlung $L_0$ nur von oben nach unten kommt.

Bringt man z.B. als Verbraucher einen so kleinen Würfel in die Mitte des Innenraums, dass er die Strahlungsintensität nicht wesentlich erniedrigt, wird er dort von allen 6 Seiten mit $L_1$ bestrahlt, während er im Freien nur von einer Seite mit $L_0$ bestrahlt würde (sofern die Strahlung senkrecht auf eine Würfelfläche auftrifft).

Daraus wird das enorme Potential ersichtlich, das sich aus dieser Lichtanreicherung ergibt. Selbstverständlich hat man beim realen Betrieb als Sauna nicht diese idealen Verhältnisse. Denn erstens sind Spiegel mit mehr als 95% Reflexion zu teuer für die hier benötigten großen Flächen und zweitens ist es ja beabsichtigt, dass die saunierenden Menschen die Strahlung absorbieren, um sich zu erwärmen. Die Isotropie der Strahlung ließe sich unter intelligenter Ausnutzung der Reflexionsgesetze für die Strahlung durch geeignete geometrische Konstruktionen weitgehend erreichen. Für die Anwendung als Sauna erscheint es aber sogar sinnvoller, auf die Isotropie zu verzichten und das Licht durch die Konstruktion in dem Bereich zu konzentrieren, wo sich die Menschen tatsächlich aufhalten.

Man sieht, dass die Verstärkung der Strahlung allein vom Verhältnis w/s abhängt. Das bedeutet, dass es keine Rolle spielt, ob man viele kleine Lichttrichter oder wenige große nebeneinander verwendet. Im Extremfall reicht sogar ein einziger Lichttrichter, der nur noch einen Spalt mit Spaltbreite s in der vollen Breite des Innenraums hat und dann gar keine Basisflächen mehr benötigt, sondern nur noch aus zwei Seitenflächen besteht.

Bei der Bestimmung des Verhältnisses s/w geht man zweckmäßigerweise folgendermaßen vor: Zunächst wird s vorgegeben, dann wird w bestimmt, und zwar derart, dass für den gewählten Winkel $\alpha$ ein möglichst großer Teil der senkrecht von oben kommenden Sonnenstrahlung insbesondere, die gesamte Strahlung, in den Innenraum eindringt. Wählt man w zu groß, dann wird der Teil der Strahlung, der weit oben am Lichttrichter, nahe der Spitze der Spiegelelemente, das erste Mal reflektiert wird, so oft reflektiert, dass er schließlich nicht mehr ins Innere gelangt, sondern wieder nach oben entweicht (siehe Strahl 17 in FIG 3). Berechnungen mit einem Simulationsprogramm führten zu folgenden optimalen Werten für w/s bei vorgegebenen Winkeln $\alpha$:

| $\alpha$ | w/s |
|---|---|
| 60° | 1,0 |
| 65° | 1,3 |
| 70° | 1,9 |
| 75° | 2,8 |
| 80° | 4,8 |
| 85° | 10,4 |
| 87,5° | 21,8 |

Diese Werte zeigen, dass sich sehr hohe Anreicherungsgrade erreichen lassen, wenn $\alpha$ nahezu 90° beträgt. Anschaulich erklären kann man dies so: Eine einfache geometrische Überlegung zeigt, dass sich der Winkel gegenüber der Horizontalen eines von oben kommenden Strahls bei jeder Reflexion an den schrägen Lichttrichterwänden um 2 * (90°- $\alpha$) verringert. Und wenn dieser Winkel durch mehrfache Reflexion negativ wird, gelangt der Strahl nicht in den Lichtkäfig, sondern entweicht aus dem Trichter nach oben.

Beispiel: $\alpha$=75°. Der Originalstrahl von oben hat den Winkel 90°. Der erste reflektierte Strahl hat dann den Winkel 90° - 2 * (90°-75°) = 60°. Der zweifach reflektierte Strahl hat noch 30° und der dritte 0°. Dies ist der "Grenzstrahl", der gerade nicht mehr ins Gefäß gelangt. Somit wird klar, dass die Zahl der möglichen Reflexionen im Lichttrichter stark wächst, wenn sich $\alpha$ an 90° annähert.

Der Nachteil dabei ist natürlich, dass die Länge der Seitenflächen des Spiegelkörpers genauso stark wächst, was dann sehr große Spiegelflächen und damit Spiegelkörper erfordert. Zudem hat man in der Praxis bei jeder Reflexion Verluste und Randeffekte, weil die Spiegel nicht ideal und auch nicht unendlich dünn sind und somit die Spiegelkörper keine perfekten Spitzen aufweisen.

Das ist für die Sonnenlichtkammer gemäß der Erfindung jedoch unerheblich, da diese keine unendlich hohe bzw. extrem hohe Strahlungsanreicherung erreichen möchte, sondern Verbraucher in Form von schwitzenden Menschen oder von Pflanzen im Innern aufweisen soll. In der Praxis ist es somit wichtig, die erzielbare Verringerung der Strahlungsverluste durch die Trichteröffnungen in Relation zur Strahlungsabsorption durch die Körper im Innenraum, beispielsweise Personen oder Einrichtungen oder Pflanzen, und dessen Wandflächen und Boden zu setzen.

Die Simulationsrechnungen zeigen auch, dass der Anteil der Strahlung, der durch die Lichttrichter in den Lichtkäfig gelangt, stark vom Einfallswinkel der Strahlung abhängt. In der Praxis bedeutet dies, dass die Lichttrichter dem Sonnenstand nachgeführt werden müssen. Hierzu können die Lichttrichter fest montiert sein und die gesamte Sonnenlichtkammer wird gedreht, oder die Sonnenlichtkammer wird stationär gebaut und die Lichttrichter bzw. Spiegelkörper sind hieran drehbar angebracht. Bei Verwendung von Lichttrichtern bzw. Spiegelkörpern der letztgenannten Art reicht in der Regel, je nach Ausrichtung, die Nachführung in einem einzigen Winkel. Wenn man einige Randverluste in Kauf nimmt und die Trichterspalten von oben nach unten ausrichtet, reicht die Nachführung des Azimuts.

Die Montage der Lichttrichter bzw. Spiegelkörper wird in der Regel im Inneren unterhalb einer entspiegelten Glasscheibe mit hohem Transmissionsvermögen erfolgen, da die Spiegel bei einer Außenmontage zu schnell verdrekken.

Für eine Überprüfung der vorgenannten Theorie durch die Praxis wurde folgender Lichtkäfig gebaut: Der Käfig war ein Würfel mit Kantenlänge 40 cm, dessen Boden und 4 Seiten jeweils aus Spiegelglas bestanden und der oben offen war. Als Lichttrichter dienten 5 Dreiecksteile der Länge 50 cm (damit sie bequem über den Käfig gelegt werden konnten), w = 9,6 cm, h = 25,9 cm, s = 2cm. Das ergab einen Winkel $\alpha$ von 80°. Als Spiegel für den Käfig und die Dreiecke wurden 2 mm dicke Silberspiegel verwendet (Verspiegelung auf der Rückseite), die laut Hersteller im sichtbaren Bereich einen Reflexionsgrad von ca. 94% haben. Gemessen wurde die Lichtanreicherung mit einem Luxmeter für sichtbares Licht, das eine (hier nicht notwendige) Anpassung an die wellenlängenabhängige Empfindlichkeit der Augen hatte. Mit dieser recht einfachen Konstruktion konnte bei Ausrichtung des Messkopfes des Luxmeters in die Einfallsrichtung der Sonnenstrahlung eine Verstärkung der Strahlung im Käfig von bis zu 2,5 gemessen werden. Bei Ausrichtung in andere Raumrichtungen war die Lichtintensität viel niedriger, im Durchschnitt wurde jedoch auch hierbei noch die einfache Lichtintensität erreicht.

Dieser Versuch zeigt, dass das der Erfindung zugrunde liegende Prinzip funktioniert und dass somit auch im deutschen Winter bei Sonnenschein in der Sonnenlichtkammer bzw. Sonnenlichtsauna eine ausreichende Lichtintensität erreicht werden kann.

Neben den vorstehend angesprochenen, eindimensionalen Lichttrichtern mit einem Spalt, der sich über die ganze Länge des Innenraums erstreckt (hier sind die Spiegelkörper beispielsweise prismenförmig ausgebildet, insbesondere mit gleichschenkligem Dreieck im Querschnitt), können auch zweidimensionale Lichttrichter verwendet werden, bei denen sich der rechteckige Querschnitt nach unten auf ein quadratisches Loch verjüngt. Diese können raumfüllend in beide horizontalen Richtungen nebeneinander platziert werden und führen zu noch geringeren Strahlungsverlusten, da die Strahlung nur noch durch ein kleines Loch und nicht mehr durch einen ganzen Spalt entweichen kann. Allerdings sind zweidimensionale Lichttrichter konstruktiv aufwendiger und erfordern eine Nachführung im Azimut und in der Elevation, d.h. in zwei Winkeln.

Es sei nochmals darauf hingewiesen, dass die Lichttrichter zwar das Sonnenlicht bündeln, so dass das Licht nicht über eine große Fläche, sondern in konzentrierter bzw. gebündelter Form, beispielsweise über einen schmalen Spalt, in das Innere der Kammer eingeleitet werden kann. Die Erhöhung der Strahlungsintensität im Inneren der Sonnenlichtkammer erfolgt jedoch nicht aufgrund dieser Lichtbündelung durch die Lichttrichter selbst, sondern durch die Verspiegelung und die damit verbundene Lichtreflexion im Innenraum der Sonnenlichtkammer. Die Lichttrichter dienen nur dazu, möglichst viel Sonnenlicht durch den Spalt in das Innere der Sonnenlichtkammer zu lenken. Hinter dem Spalt im Inneren der Kammer fächert sich das Licht sofort wieder auf, die Lichtkonzentrierung in der Sonnenlichtkammer wird nur durch die weitgehende Verspiegelung der Innenflächen der Kammer erreicht. Die Funktion der Lichttrichter liegt somit darin, die für den Lichteintritt erforderliche Fläche deutlich zu verringern und somit eine teilweise Verspiegelung der Innenseite der Lichteintrittsseite der Sonnenlichtkammer zu ermöglichen, wodurch der Lichtaustritt erschwert und die Lichtkonzentration im Inneren der Sonnenlichtkammer erhöht wird.

Es ist auch möglich, mit einer Anordnung aus Sammellinsen die Sonnenstrahlung auf einen kleinen Bereich zu fokussieren und dann mit Glasfasern in eine verspiegelte Kammer bzw. Sauna im Innern eines Gebäudes zu leiten. Dies funktioniert vergleichbar mit den vorstehend angesprochenen zweidimensionalen Lichttrichtern, indem am engen Trichterloch die Strahlung in eine Glasfaserleitung eingespeist und zur Kammer bzw. Sauna geleitet wird. Der Vorteil ist hierbei die Trennung des Lichtfängers von der Sauna. Außerdem wäre die einzufangende Lichtmenge nicht mehr von der Saunagröße abhängig. Diese Ideen werden auch unabhängig von den vorstehenden Ausführungen beansprucht.

Es gibt auch noch andere Methoden, um die Strahlungsintensität im Innenraum der Sonnenlichtkammer bzw. Sonnenlichtsauna zu erhöhen. Beispielsweise kann mit großen externen Spiegeln zusätzlich Licht in den Innenraum gelenkt werden. Dasselbe ist möglich mit künstlichen Strahlungsquellen.

Bei der erfindungsgemäßen Sonnenlichtkammer können hohe Strahlungsintensitäten auftreten, insbesondere wenn sich keine Personen und/oder sonstigen absorbierenden Gegenstände im Innenraum befinden. Aus diesem Grund ist es zweckmäßig, darauf zu achten, dass die Sauna keine Strahlungsleckagen aufweist, die Strahlung unkontrolliert von innen nach außen gelangen lassen. Hätte z.B. eine Spiegelwand eine schadhafte Stelle, durch die die Strahlung ungehindert austreten könnte, bestünde die Gefahr, dass sich eine Person, die sich die Stelle genauer ansehen möchte, sehr intensive Strahlung ins Auge bekommt und trotz des reflexartigen Lidschlusses Schaden nehmen könnte. Solche Unfälle gelten allerdings als sehr selten, weil in aller Regel der Lidschluss-Reflex des Auges schnell genug erfolgt.

Ein Strahlenleck, das nicht vermeidbar ist, ist die Tür. Durch eine Drehung der verspiegelten Tür wird Strahlung in einen weiten Bereich der Umgebung gestreut. Wenn noch kein Benutzer in der Sauna ist, könnte die Intensität auch ein Mehrfaches der normalen Intensität der Sonnenstrahlung betragen. Es ist daher zweckmäßig, zu vermeiden, dass diese Strahlung unkontrolliert in die Umgebung gelangt. Mögliche Gefahren sind: Geblendete Verkehrsteilnehmer, Augenschäden durch zu hohe Strahlungsintensität, usw.

Für eine Lüftung und den Anschluss von Geräten können Durchführungen durch die Wände (und/oder ein Gerüst, das die Wände trägt) nötig sein. Während sich Durchführungen für Kabel einfach abdichten lassen, könnten Löcher für die Lüftung Strahlung nach außen gelangen lassen. Auch dies sollte vermieden werden.

Vorteilhafte Weiterbildungen der Erfindung sehen einen Schutz vor zu hohen Strahlungsintensitäten und die Nutzung von überschüssiger Strahlung vor. Hierzu folgende Anmerkungen:

Bei klarem Himmel kommen bei senkrechtem Sonnenstand 75% der Solarkonstante ($1367 \text{ W/m}^2 = 0{,}1367 \text{ W/cm}^2$) auf die Erdoberfläche (Meereshöhe). Bei schrägem Einfall ist dies entsprechend dem längeren Weg der Strahlung durch die Atmosphäre zu korrigieren. Der Grenzwert für die maximale Hautbestrahlung mit Strahlung im Bereich von 380 nm bis 3000 nm liegt bei ca. $2 \text{ W/cm}^2$ (ungefähr die zwanzigfache maximale Strahlungsintensität auf der Erdoberfläche). Dies gilt streng aber nur für Zeiten kleiner als 10 Sekunden. Für größere Zeiten gibt es keine Empfehlungen, stattdessen wird die Schmerzgrenze als noch unschädlich angenommen.

Besonders kritisch sind UV- und ferne Infrarot-Strahlung, da diese nur eine sehr kleine Eindringtiefe in die Haut haben und praktisch schon vollständig von der Epidermis (abgestorbene Außenhaut) absorbiert bzw. reflektiert werden. Sichtbares Licht und nahe Infrarot-Strahlung (das ist der größte Teil des Sonnenspektrums) hingegen haben eine größere Eindringtiefe und daher verteilt sich die in die Haut dringende Strahlung auf ein größeres Volumen, wodurch die Gefahr einer lokalen Überhitzung entsprechend kleiner ist.

Zum Vergleich: Laser induzierte Thermotherapie wird typischerweise mit 6 bis $12 \text{ W/cm}^2$ Dauerleistung durchgeführt. Das Ziel dabei ist eine Koagulation im Gewebe durch eine leichte Überhitzung. In der Sonnenlichtkammer bzw. Sonnenlichtsauna sollten daher die Strahlungsintensitäten deutlich unter diesen Werten bleiben.

Wenn daher beispielsweise in großen Sonnenlichtsaunen die Gefahr besteht, dass die Strahlungsintensität zu groß wird, kann ein Mechanismus vorgesehen werden, der die Intensität begrenzt. Hierzu sollte eine Messung der Strahlungsintensität im Innenraum erfolgen. Sinnvoll ist beispielsweise eine Anzeige der Messwerte, die man von innen und von außen eingesehen werden kann. Es gibt zahlreiche geeigneten Maßnahmen für eine Begrenzung der Strahlungsintensität. Hier einige Beispiele:

a) Manuell anzubringende Beschattung von außen.

b) Verwendung von Glasscheiben, deren Transmissionsvermögen von außen nach innen über eine elektrische Spannung gesteuert werden kann.

c) Eine elegante Lösung ist die Verbindung von Strahlungsbegrenzung und gleichzeitiger Nutzung der überschüssigen Strahlung. Dies kann beispielsweise durch das automatische Einfahren von Konzentratorzellen geschehen. Das sind Solarzellen, die für hohe Lichtintensitäten entwickelt wurden. Damit lässt sich die überschüssige Strahlung in Strom verwandeln.

d) Weiter ist es möglich, einen dunklen Stein mit hoher Wärmekapazität in die Sonnenlichtkammer bzw. Sonnenlichtsauna zu stellen. Dieser kann dann beispielsweise am Abend, wenn er warm ist, mit in ein Gebäude (z.B. Wohnzimmer) genommen werden, um dort seine Wärme abzugeben.

e) Auch eine automatische Steuerung ist möglich, indem um den vorgenannten dunklen Stein eine spiegelnde Abdeckung angebracht wird, die bei zu hoher Strahlungsintensität geöffnet wird. Diese Abdeckung ist auch als warmer Sitzplatz für Saunagäste verwendbar.

f) Die überschüssige Strahlung kann auch über Lichtleiter (z.B. Glasfaser) in ein Gebäude geleitet werden, wo es dann als natürliches Licht genutzt oder in Wärme umgewandelt werden kann. Konstruktiv lässt sich dies beispielsweise lösen, indem im Boden ein Loch angeordnet wird, in dem die Lichtleiter beginnen. Ist keine Person in der Sauna, wird dieses Loch geöffnet und die Strahlung gelangt durch die Lichtleiter an den gewünschten Ort. Ist jemand in der Sauna, wird das Loch mit einem verspiegelten Deckel verschlossen.

Die vorgenannten Lösungen mit einer Nutzung der überschüssigen Strahlung sind besonders interessant, wenn die Kammer bzw. Sauna nur wenig genutzt wird. Befindet sich eine Sonnenlichtsauna beispielsweise für den Privatgebrauch in einem Garten, so ergibt sich dadurch noch einen zusätzlicher Nutzen für den Eigentümer.

Bei der Berechnung der notwendigen Größe der Sonnenlichtkammer bzw. Sonnenlichtsauna sollten folgende Punkte berücksichtigt werden: Erwachsene Menschen haben durchschnittlich eine Hautoberfläche von etwa HO = 2 qm. Im Idealfall gibt es keine Strahlungsverluste und die Benutzer absorbieren die ganze einfallende Strahlung. Hat man nun eine Lichteinfangfläche von LF = 10 qm mit senkrechtem Lichteinfall, so hätte man bei einer Personenzahl PZ = 2 eine Verstärkung der Strahlung um den Faktor V = LF / (PZ * HO) = 2.5. Trifft das Licht gemäß dem aktuellen Sonnenstand unter dem Winkel x auf die Glasoberfläche, ergibt sich folgende Formel:

$$V = \sin(x) * LF / (PZ * HO) \qquad (x = 90° \text{ bei senkrechtem Einfall})$$

Für Privatanwender ist es empfehlenswert, die Sauna mindestens so groß zu wählen, dass auch noch im Winter zumindest in der Mittagszeit eine ausreichende Strahlungsstärke erreicht wird.

Die Messung der Lufttemperatur im Inneren der Sauna wäre zwar wünschenswert, dürfte aber wegen der hohen Strahlungsintensität schwierig sein. Außerdem fühlt die Haut, wenn sie durch starke Strahlung aufgeheizt wird, von der Lufttemperatur nur wenig.

Ob eine künstliche Lüftung nötig ist, hängt von der genauen Konstruktion und der Dauer der Nutzung ab. Je nach Bauart kann es sein, dass genügend Fugen vorhanden sind, die eine ausreichende natürliche Lüftung ermöglichen. Ist dies nicht der Fall und reicht die Lüftung durch die Türbewegungen nicht aus, sollte für eine künstliche Belüftung gesorgt werden. Andernfalls besteht die Gefahr, dass die Qualität der Innenluft abnimmt und die Lichttrichter und Wände durch die ausgeschwitzte Feuchtigkeit beschlagen. Dies muss jedoch nicht zu einer deutlichen Verringerung der Strahlungsintensität führen. Auf der einen Seite absorbiert Wasser bzw. Wasserdampf die Infrarot-Strahlung (das sichtbare Licht weniger). Auf der anderen Seite verdampfen aber gerade dadurch die Wassertröpfchen, die sich auf der Glasoberfläche niederschlagen, noch im Entstehen. Dieses dynamische Gleichgewicht hängt sehr stark von der Außentemperatur bzw. von der Temperaturdifferenz zwischen innen und außen ab.

Bei sehr kalten Außentemperaturen könnte es angebracht sein, die Frischluftzufuhr vorzuwärmen, damit erstens die Abluft mehr Feuchtigkeit aufnehmen und abführen kann und zweitens die Gefahr einer Erkältung für die Benutzer nicht zu groß wird, da deren Haut zwar durch die Strahlung erwärmt wird, aber die Luft, die sie atmen, zu kalt sein könnte.

Gegebenenfalls kann ein Beschlagen der Spiegel auch dadurch vermieden werden, dass Spiegelglas verwendet wird, das durch eine geeignete Beschichtung oder Behandlung vor Beschlagen geschützt ist.

Bei einer Lüftung sollte beachtet werden, dass durch die Löcher, die für die Luftzufuhr bzw. Abfuhr nötig sind, erstens keine Strahlung nach Außen gelangt (Schutz der Außenwelt vor zu intensiver Strahlung) und zweitens die Strahlungsverluste minimal sind. Werden für ein Gerüst der Kammer bzw. Sauna Materialien verwendet, die innen zumindest teilweise hohl sind (z.B. Metallrohre), so können diese Hohlräume von außen an eine Warmluftzufuhr angeschlossen werden und von innen können viele sehr kleine Löcher zum Hohlraum hin gebohrt werden. Damit lässt sich eine zugfreie, gleichmäßig verteilte Warmluftzufuhr realisieren.

Dort, wo eine Inneneinrichtung notwendig erscheint, sollte darauf geachtet werden, dass von dieser möglichst wenig Strahlung absorbiert wird. Dies kann erreicht werden, indem die Außenseiten der Gegenstände verspiegelt werden, oder indem möglichst helle (bzw. durchsichtige) Materialien verwendet werden. Diese Gegenstände sollten ferner für die auftretenden Strahlungsintensitäten geeignet sein, beispielsweise könnten sich bereits kleine dunkle Stellen an diesen Gegenständen bei den hohen Strahlungsintensitäten, die insbesondere dann auftreten, wenn sich keine Person in der Kammer bzw. Sauna aufhält, sehr stark erwärmen. Geeignet erscheinen: Sitzmöbel aus Glas, Luftmatratzen aus durchsichtigem Plastik. Wenn Saunahandtücher verwendet werden, sollten diese weiß und nicht übermäßig groß sein.

Um die nötigen Reinigungsarbeiten zu minimieren, sollte schon bei der Konstruktion darauf geachtet werden, dass möglichst wenig Schmutzeintrag stattfinden kann. Das betrifft sowohl natürlichen Dreck, der z.B. durch den Wind über undichte Fugen ins Innere gelangen könnte, als auch die Benutzer. Gerade wenn der Boden aus Glas ist, sollte darauf geachtet werden, dass möglichst wenig Schmutz über die Füße hineingetragen wird. Ein Fußbad vor dem Eintritt könnte sinnvoll sein, ebenso die Verwendung von weißen Stoff-Pantoletten (auch aus Hygienegründen).

Auch das Eindringen von Tieren aller Art, insbesondere von Insekten, sollte vermieden werden, da sie die Innenwände und Spiegelflächen der Glaswände beschmutzen und den Benutzern lästig werden könnten.

In einer Ausführungsvariante ist eine Konstruktion ohne spiegelnden Boden vorgesehen. Wenn der Standort genügend Sonne bietet, kann beim Boden auf Spiegelglas verzichtet werden, da dieses ohnehin recht empfindlich gegenüber Kratzern ist und bei einem mangelhaften Fundament auch brechen kann. Am Sandstrand kann der Boden beispielsweise aus sehr hellem Sand (mit hoher Albedo) bestehen. Oder es wird dunkler Sand verwendet, wenn der Boden absichtlich erwärmt werden soll. In diesem Fall kann man beispielsweise auch im Winter in der Türkei am Strand sonnenbaden. In Saunaanlagen können Böden aus sehr hellem, pflegeleichtem Material sinnvoll sein. Auch ist die Verwendung eines Spiegels nicht aus Glas, sondern aus einem anderen Material, möglich.

Ein wesentlicher Vorteil der erfindungsgemäßen Sonnenlichtsauna im Vergleich zu bekannten Saunen liegt in der Autonomie. Im einfachsten Fall, d.h. ohne jegliche elektronische Zusatzausstattung, ist die Sonnenlichtsauna vom elektrischen Strom und anderen Materialien zur Wärmeerzeugung unabhängig und kann prinzipiell überall aufgestellt werden, wo es genügend Sonne gibt: in der Wüste, am Strand, auf einem Berg, im Privatgarten, in Saunaanlagen, usw. Denkbar ist sogar eine mobile Konstruktion, z.B. auf einem Kfz-Anhänger.

Ein weiterer Vorteil liegt in der sehr hohen Energieeffizienz. Wenn genügend Menschen in der Sauna sind und auf eine künstliche Strahlungsreduktion verzichtet werden kann, geht der allergrößte Teil der gesammelten Sonnenstrahlung in die Körper der Menschen. Im Vergleich zu anderen Solarsaunen ist dies ein großer Vorteil, weil dort viel Energie in die Aufheizung des Gebäudes, der Einrichtung und der Luft gesteckt wird.

Ein Vorteil liegt auch in minimalen Betriebskosten. Die Betriebskosten der erfindungsgemäßen Sonnenlichtsauna sind sehr günstig, da keine künstliche Heizung nötig ist. Reinigungsarbeiten müssen jedoch wie bei jeder Sauna einkalkuliert werden. Alle Flächen, an denen Strahlung reflektiert werden soll, sollten möglichst sauber gehalten werden, um die Strahlungsabsorption möglichst gering zu halten. Bei Ausstattung mit Lichtstärkemessgeräten, Lichtstärkereglern, Überwachungskameras, etc. sollten diese Geräte regelmäßig gewartet werden.

Als Nachteil könnte angeführt werden, dass die Sonnenlichtsauna nur funktioniert, wenn die Sonne scheint. Gegebenenfalls reicht aber auch das gesammelte Tagesstreulicht bei bedecktem Himmel für einen Saunaeffekt aus. Wenn die Sauna aus irgendeinem Grund zuwenig Sonnenstrahlung erhält (Schatten durch größer gewordene Bäume, Ansteigen der Besucherzahlen, zuwenig Platz für größere Sauna, usw.), kann auch durch zusätzliche Vorrichtungen mehr Sonnenlicht in die Sauna eingestrahlt werden. Beispielsweise sind dazu Spiegel geeignet, die das Sonnenlicht in die Sauna hineinspiegeln. Meist benötigt man dann aber eine Nachführvorrichtung, um die Spiegelausrichtung dem jeweiligen Sonnenstand anzupassen.

Grundsätzlich ist es auch möglich, durch künstliche Strahlung und/oder Heizung die Sauna auch in Zeiten geringen Sonnenscheins nutzbar zu machen. Besonders einfach ist es, außerhalb der Sonnenlichtsauna erzeugte Licht- bzw. Wärmestrahlung auf die Sauna zu richten und damit für Strahlung in der Sauna zu sorgen. Eine dauerhafte Heizung im Inneren hätte den Nachteil, dass das Heizgerät einen Teil der Sonnenstrahlung absorbieren würde. Der Vorteil wäre jedoch, dass mit einer Innenheizung das Beschlagen der Spiegel und Wände verringert werden könnte. Bei einer Warmluftzufuhr von außen besteht (gerade in Saunaanlagen) hingegen die Gefahr, dass damit auch Feuchtigkeit ins Innere transportiert wird, wodurch die Innenteile schneller beschlagen könnten.

Außerdem kann es erforderlich sein, die Zahl der Menschen in der Sauna zu beschränken, damit diese sich nicht gegenseitig die Strahlung wegnehmen. In öffentlichen Saunaanlagen könnte deshalb eine Zugangskontrolle für die Sonnenlichtsauna zweckmäßig sein, die beispielsweise über die aktuelle Lichtstärke in der Sauna gesteuert werden kann.

Ein weiterer Vorteil der erfindungsgemäßen Sonnenlichtkammer bzw. Sonnenlichtsauna liegt in dem geringen Wartungsaufwand. Der zugrundeliegende Lichtkäfig ist bei geeigneter Konstruktion sehr wartungsarm. Von außen kann er mit einem Hochdruckreiniger abgespritzt werden. Im Inneren ist je nach Nutzungsart eine regelmäßige Reinigung des Bodens und der Inneneinrichtung nötig. Zu beachten ist, dass Schweißtropfen nicht wie in einer normalen Sauna durch die heiße Luft verdampft und auch nicht vom Holz aufgenommen werden, sondern auf den kühlen Flächen liegen bleiben. Unter Umständen verdampfen sie jedoch aufgrund von Erwärmung durch Absorption von Strahlung.

Vorteilhaft sind auch die Effekte der Sonnenlichtsauna auf den Körper. Anzuführen ist zum einen der thermische Sauna-Effekt. Die Unterschiede zu anderen Saunen sind:

a) Aufheizung des Körpers nicht durch heiße Luft, sondern direkt durch die Sonnenstrahlung. Die Luft kann unter Umständen sogar recht kühl sein, sofern keine thermische Isolation vorgesehen ist.

b) Da der größte Teil der Sonnenstrahlung eine verhältnismäßig große mittlere Eindringtiefe in die Haut hat, kann die Aufheizung des ganzen Körpers entweder schneller erfolgen als bei einer normalen Sauna (bei hoher Lichtintensität) oder schonender, weil die äußere Hautschicht nicht so heiß wird (bei niedriger Lichtintensität). In jedem Fall erscheint es günstig, wenn durch das Eindringen der Strahlung in die Haut ein größeres Volumen erwärmt wird, weil dadurch lokale Überhitzungen vermieden werden. Außerdem sind die tiefer liegenden Hautschichten durchblutet und somit kann die Wärme sehr rasch abtransportiert werden. Im Vergleich dazu wird bei den üblichen heißen Saunen durch den Kontakt mit der heißen Luft zuerst einmal die äußere Hautschicht überhitzt und die Wärme muss dann in die tieferen Hautschichten wandern, von wo sie mit dem Blut abtransportiert und im ganzen Körper verteilt wird. Das gleiche passiert auch bei den Infrarot-Saunen, weil die dort erzeugte Infrarot-Strahlung praktisch vollständig in der Epidermis absorbiert wird.

c) Im Gegensatz zum Sonnenbad an der freien Luft wird in der Sonnenlichtsauna wegen der Spiegel der größte Teil der Körperoberfläche gleichzeitig bestrahlt, was mindestens einer Verdoppelung der bestrahlten Oberfläche entspricht.

d) In der Grundform der Sonnenlichtsauna wird nur natürliche Strahlung verwendet, keine künstliche Wärme.

Zum anderen ist auf die Lichttherapie in der Sonnenlichtsauna zu verweisen. Die Naturmedizin weiß um die Wirksamkeit von Licht im Körper. Die Sonnenlichtsauna bietet die Möglichkeit zu einer intensiven Lichttherapie am ganzen Körper mit natürlichem Sonnenlicht. Um die dadurch möglichen positiven feinstofflichen Effekte nicht ins Gegenteil zu verkehren, ist allerdings eine sorgfältige Auswahl der Materialien nötig. Denn das Licht wird ja einige Male gespiegelt und kann dabei feinstofflich verunreinigt werden. Aus diesem Grund könnte es vorteilhaft sein, als Spiegelglas richtiges Glas zu verwenden und kein Plexiglas und als Metallschicht für den Spiegeleffekt wären Gold oder Silber die optimale Wahl.

Ein großer Vorteil der Sonnenlichtsauna ist, dass es durch die Verstärkung der Strahlung selbst in Deutschland auch noch im Winter möglich ist, die Sauna zu nutzen, sofern sie groß genug gebaut wird. Da der Körper im Winter ansonsten wegen der Kleidung sehr wenig Sonnenstrahlung abbekommt, ist dies eine sehr günstige Möglichkeit, auch im Winter Licht zu tanken.

Ein weiterer Vorteil der Sonnenlichtkammer bzw. Sonnenlichtsauna ist die Abschirmung von Elektrosmog. Wenn alle Wände aus Spiegelglas sind und die Metallschicht des Spiegelglases mit einem metallenen Gerüst verbunden wird, ergibt sich einen sehr guten Faradayschen Käfig, der große Teile des Elektrosmogs abschirmt.

Durch die Wahl der Form können gegebenenfalls noch andere Naturkräfte aktiviert und die Sonnenlichtsauna auch in Zeiten ohne Sonne alternativ genutzt werden. Als Beispiele seien eine oktogonale Form für Orgon-Anwendungen und eine Pyramidenform für spirituelle Sitzungen genannt.

Anzumerken ist, dass die Sonnenlichtsauna gemäß der Erfindung die Beachtung bestimmter Vorsichtsmaßnahmen mit sich bringen kann. So sollte darauf geachtet werden, dass die Haare nicht zu heiß werden, eventuell sollte mit Wasser gekühlt werden. Für die Kühlung von Körperhaaren müsste gegebenenfalls eine Dusche installiert werden. Bei hoher Lichtintensität sind gegebenenfalls die Augen zu schützen, beispielsweise durch Schutzbrillen, wie sie bei UV-Bräunungsgeräten verwendet werden. Möglicherweise kann die Gefahr von Blitzeinschlägen bestehen. In diesem Fall sollte auf eine Nutzung der Sauna verzichtet werden.

Eine konkrete Form einer erfindungsgemäßen Sonnenlichtsauna wurde bereits anhand FIG 1 und FIG 4 erläutert. Hierbei handelt es sich um eine Pyramide mit quadratischer Grundfläche. Das Verhältnis Höhe zu Breite an der Basis kann entsprechend dem Goldenen Schnitt 1,618 betragen. Der Goldene Schnitt besagt, dass der Mensch die Aufteilung einer Fläche oder Strecke im Verhältnis von etwa 3 : 5 bzw. genauer 1 (= b) : 1,618 (= a) als besonders harmonisch empfindet. Ein asymmetrisches Teilungsverhältnis, das den Prinzipien des Goldenen Schnittes folgt, wird vom Betrachter auch als natürlich, das heißt in Übereinstimmung mit der Natur, bewertet.

$$h = l*\sin(x), \quad b/2 = l*\cos(x) \implies 2h / b = \tan(x) \implies x = 72{,}83°$$

Das ergibt eine Steilheit der Pyramidenwände von 72,83°.

Neben den wissenschaftlich umstrittenen Pyramidenkräften hat diese Form den Vorteil, dass es kein Dach gibt (schwer zu reinigen, Dreck bleibt liegen, ebenso Schnee). Die steilen Wände können einfach durch Abspritzen mit Wasser gereinigt werden und Schnee rutscht von selbst nach unten.

Die Steilheit der Wände von ungefähr 72° bis 73° hat dazu noch den Vorteil, dass im Winter bei niedrigem Sonnenstand das Licht fast senkrecht einfallen kann und somit optimal ausgenutzt wird, während im Sommer die Ausnutzung geringer ist, was aber durch die intensivere Strahlung mehr als ausgeglichen wird. Zu bedenken ist auch, dass die Basisfläche im Innenraum umso besser ausgenutzt werden kann, je steiler die Wände sind.

In FIG 1 sind die Lichttrichter auf der Vorderseite nur angedeutet. Normalerweise sind sie nicht auf der Seite mit der Türe und erstrecken sich über die ganze Seite der Pyramide. Der Sonneneinfall ist von vorn her anzunehmen. Auf der Seite mit dem Lichttrichter wird als Wand kein Spiegelglas verwendet, sondern normales Glas mit möglichst hoher Transmission.

Der Boden besteht aus einer nach innen hin verspiegelten, relativ dicken, möglichst bruchfesten Glasplatte, die auf einem drehbaren (zur automatischen oder manuellen Azimut-Nachführung), ebenen Fundament liegt. Möglich sind z.B. ein Betonfundament oder eine dicke Eisenplatte. Wichtig beim Fundament ist, dass es sich nicht in sich verformt, weil sonst die darüber liegende Glasplatte schnell brechen kann. Aus diesem Grund kann es auch sinnvoll sein, mehrere kleinere Glasplatten als Boden zu verwenden. Zur mechanischen Dämpfung und als Kratzschutz für die Glasplatte kann zwischen Fundament und Glasplatte noch eine Gummiplatte gelegt werden.

Alternativ könnte als Boden auch eine pflegeleichte, elastische, sehr weiße Plastikmatte verwendet werden.

Die Pyramide wird über dem Boden mit Hilfe eines Gerüstes errichtet, in das dann die Spiegelglasscheiben eingesetzt werden. Die Teile des Gerüsts, deren Oberfläche im Inneren der Pyramide sichtbar ist, sollten möglichst wenig Strahlung (Licht und nahes Infrarot) absorbieren. Wenn die Absorption durch diese Teile zu groß ist, kann diese durch Aufbringung einer Spiegelschicht, beispielsweise Silberfolie, verringert werden. Das Gerüst sollte möglichst schlank sein, damit es nicht unnötig viel Sonnenstrahlung abhält. Außerdem ist es den Witterungseinflüssen ausgesetzt und von innen her ständig mit Feuchtigkeit (Schweiß) konfrontiert. Empfehlenswert erscheint deshalb die Verwendung eines absolut rostfreien Edelstahls.

Als Wände für die Pyramide soll Spiegelglas verwendet werden. Die Dicke ist den mechanischen Beanspruchungen anzupassen (Witterung, Bälle von spielenden Kindern, usw.). Die Verspiegelung sollte durch die Aufbringung einer Gold- oder Silberschicht erfolgen (andere übliche Spiegelmetalle wie Aluminium sind zwar möglich, haben aber einen wesentlich kleineren Reflexionskoeffizienten.)

Bei kleinen Pyramiden kann man evt. jede der 4 Seiten aus einem einzigen dreieckigen Glasstück machen. Bei größeren Pyramiden sollte hingegen jede Seite aus mehreren Glasteilen zusammengesetzt werden.

Für die Befestigung der Glasscheiben am Gerüst gibt es unterschiedliche Möglichkeiten. Einige davon sind:

a) Man verwendet für die Ecken des Gerüsts von den Ecken der Basis schräg nach oben zur Spitze ein T-Profil, wobei der Mittelsteg nach außen schaut. Unten an der Basis verbindet man diese Träger mit einem Winkelstück. Wenn die Seiten aus mehreren Scheiben bestehen, baut man noch Querstege (T-Profil) ein. Die Verbindung der einzelnen Metallteile sollte so sein, dass man sie auch leicht wieder lösen kann, aber auch, dass überstehende Teile nicht das Einsetzen der Glasscheiben behindern. Gegebenenfalls ist zu beachten, dass bei Schrauben die Gefahr besteht, dass sie durch ihre spiralförmige Windung einen Einfluss auf die feinstofflichen Energien nehmen können. Deshalb wären z.B. Steckverbindungen vorzuziehen. In dieses Gerüst kann man dann einfach die Glasscheiben einlegen, die aufgrund der Neigung der Seitenwände grundsätzlich von selbst halten. Um jedoch eine Beschädigung bzw. ein Abheben der Scheiben bei Sturm zu verhindern, sollten auch die Glasscheiben zumindest noch leicht fixiert werden, bevorzugt jedoch derart, dass die Scheiben leicht zu wechseln sind. Zur Vermeidung unangenehmer Geräusche sollte zwischen Metall und Glas ein Dämpfungsmaterial vorgesehen werden. Bei der Auswahl dieses Materials ist zu beachten, dass es möglichst dünn ist, große Strahlungsintensitäten von innen aushält, der Witterung von außen standhält und möglichst wenig Strahlung absorbiert. Wenn man von diesem Material einen gleichmäßig dicken, durchgehenden Streifen verwendet, kann man eine relativ gute Abdichtung nach außen erreichen. Wenn man das Material nur punktuell aufbringt, hätte man Lüftungsschlitze für eine selbständige Belüftung der Pyramide. Allerdings besteht dann auch die Gefahr, dass durch diese Schlitze Schmutz, Pollen, Wasser, Insekten und Wind eindringen und Lichtstrahlen hoher Intensität nach außen gelangen können.

b) Die obige Lösung hat den Nachteil, dass vom T-Profil ein Teil nach innen schaut. Wenn es einen hohen Reflexionsgrad hat, sollte dies nicht allzu nachteilig sein. Optimal ist es jedoch, wenn die Glasscheiben von ihrer Rückseite her so am Gerüst befestigt werden, dass sie bis auf die unvermeidlichen Stoßkanten eine durchgehende Spiegelwand erzeugen. Dazu könnte man z.B. die Glasscheiben am Gerüst festkleben. Dabei ist jedoch auf ausreichende Flexibilität zu achten, um ein Springen der Scheiben bei Bewegungen des Gerüstes zu vermeiden.

Bei der Spiegelauswahl ist auf einen möglichst hohen Reflexionsgrad zu achten. Einfache metall-beschichtete Glasspiegel haben meist eine Absorption von 5% bei der Reflexion. Es gibt aber beispielsweise für den Einsatz in Lasern auch dielektrische Spiegel, die keine Absorption aufweisen. Gegebenenfalls kommt auch die Verwendung von Isolierglasscheiben in Betracht, wobei die innere Scheibe der Spiegel ist. Damit liegt die Metallschicht im geschützten Inneren des Isolierglases und die äußere Scheibe kann als reine Glasscheibe gut gereinigt werden. Die Vorteile sind: Bessere thermische Isolation des Pyramiden-Innenraums und weniger beschlagenen Scheiben. Nachteilig könnten die höheren Kosten und das größere Gewicht, das ein stärkeres Gerüst erfordert, sein.

Zur Verstärkung und Erweiterung des feinstofflichen Energiespektrums kann man noch Edelsteine bzw. Kristalle in die Pyramide integrieren. Am Besten geeignet dafür ist der Bereich in der Pyramidenspitze (vgl. FIG 4), entweder knapp unterhalb oder knapp darüber. Denkbar ist auch eine Konstruktion, bei der das Metallgerüst gar nicht selber die Spitze bildet, sondern oben eine Aussparung aufweist, in die dann der Stein gelegt wird. Um eine vernünftige Abdichtung zu erreichen, braucht man eine Spezialkonstruktion und einen witterungsbeständigen Stein, der idealer Weise die Form einer Doppelpyramide haben sollte, wovon der obere Teil über die große Pyramide hinaus ragt und somit deren Spitze bildet. Der untere Teil ragt in die große Pyramide hinein und leitet die feinstoffliche Energie ins Innere, wo sie durch die große Pyramide verstärkt wird.

Wenn die Pyramide im Freien steht, sollte ein Blitzschutz vorgesehen werden, zumal das Metallgerüst sehr anziehend auf Blitze wirken kann. Zumindest sollte das Gerüst gut geerdet sein und der Aufenthalt in oder in der Nähe der Pyramide sollte bei Gewitter vermieden werden. Bei Verwendung eines Edelsteins als Pyramidenspitze besteht die Gefahr, dass dieser Stein bei einem Blitzschlag zerspringen kann und die Splitter mit einiger Wucht durch die Gegend fliegen können. Deswegen sollte der Stein bei Blitzschlaggefahr entfernt oder mit einer Metallkappe, die mit dem Gerüst verbunden ist, abgedeckt werden.

Insgesamt wird mit der Erfindung somit eine Sonnenlichtkammer vorgeschlagen, bei der durch die Kombination von Lichttrichter bzw. Konzentrieranordnung zum Lichteinfang einerseits und verspiegelten Wänden andererseits eine Erhöhung der Lichtkonzentration im ganzen Volumen der Sonnenlichtkammer erreicht wird, nicht nur an einem kleinen "Brennfleck" bzw. in einem eng begrenzten Bereich.

In der Ausführungsvariante als Sauna ermöglicht die erfindungsgemäße Sonnenlichtkammer, dass saunierende Personen von allen Seiten mit Sonnenlicht bestrahlt werden. Dies ermöglicht eine Anwendung der Sauna auch in kühlen Jahreszeiten. Die Erwärmung der Personen erfolgt direkt durch das Auftreffen der Sonnenstrahlung auf die Haut, nicht durch heiße Luft in der Sauna. Durch das Eindringen von sichtbarem Licht in die Haut erfolgt die Erwärmung schnell und schonend. Von Vorteil ist auch, dass die Bestrahlung der Saunierenden mit natürlichem Sonnenlicht erfolgt. Durch das konzentrierte Sonnenlicht von allen Seiten wird ferner eine intensive Lichttherapie möglich.

Insgesamt benötigt die erfindungsgemäße Sonnenlichtkammer, auch in der Ausführungsvariante als Sauna, nur Sonnenlicht und keine künstlich erzeugte Energie. Dadurch sind die Betriebskosten minimal.

**Bezugszeichenliste**

1      Sonnenlichtkammer, Sonnenlichtsauna

2      Innenraum der Sonnenlichtkammer 1

3      erste Wand- und/oder Deckenfläche

4      zweite Wand- und/oder Deckenfläche

5      Lichttrichter

6      engste Stelle des Lichttrichters 5

7      Randfläche des Lichttrichters 5

8      Seitenfläche des Spiegelkörpers 12

9      Basisfläche des Spiegelkörpers 12

10     Tür

11     Boden der Sonnenlichtkammer 1

12     Spiegelkörper

13     Pyramidenspitze

14     Halterung

15     Edelstein, Kristall

16     Sonnenstrahl

17     Sonnenstrahl

18     Traggerüst

s      Trichterbreite an der engsten Stelle 6 des Lichttrichters 5

w      Basisbreite der Basisfläche 9 des Spiegelkörpers 12

$\alpha$      Basiswinkel des Spiegelkörpers 12

**EP 2 167 009 B1**

**Patentansprüche**

1. Anordnung umfassend

   a) eine Sonnenlichtsauna (1) oder ein Gewächshaus

   a1) mit einen Innenraum (2) umschließenden Wand- und/oder Deckenflächen (3, 4) und einem Boden (11),
   a2) wobei mindestens eine erste (3) der Wand- und/oder Deckenflächen und/oder der Boden (11) zum Innenraum hin zumindest überwiegend gerichtet und/oder diffus reflektierend für sichtbares Licht und/oder infrarote Strahlung und/oder UV-Strahlung ausgebildet ist,
   a3) wobei mindestens eine zweite (4) der Wand- und/oder Deckenflächen zumindest abschnittsweise transparent für sichtbares Licht und/oder infrarote Strahlung und/oder UV-Strahlung ausgebildet ist,
   a4) wobei an und/oder in der oder den zweiten Wand- und/oder Deckenflächen (4) ein oder mehrere Lichttrichter (5) derart angeordnet oder ausgebildet sind, dass eine engste Stelle (6) des Lichttrichters (5) dem Innenraum (2) zugewandt ist,
   a5) wobei die Randflächen (7) der Lichttrichter (5) von gerichtet reflektierenden Seitenflächen (8) zueinander benachbarter Spiegelkörper (12) gebildet sind,
   a6) wobei die Spiegelkörper (12) dem Innenraum zugewandte Basisflächen (9) aufweisen, zwischen denen die engsten Stellen (6) der Lichttrichter (5) ausgebildet sind,
   a7) wobei die Basisflächen (9) zum Innenraum (2) hin zumindest überwiegend gerichtet und/oder diffus reflektierend für sichtbares Licht und/oder infrarote Strahlung und/oder UV-Strahlung ausgebildet sind, und

   b) mindestens eine Messeinrichtung zur Ermittlung der Strahlungsintensität im Innenraum (2) der Sonnenlichtsauna (1) oder des Gewächshauses, und
   c) mindestens eine Anzeige für die durch die Messeinrichtung ermittelten Messwerte außerhalb der Sonnenlichtsauna (1) oder des Gewächshauses.

2. Anordnung nach Anspruch 1,
   **dadurch gekennzeichnet, dass**
   die Anordnung mindestens eine Vorrichtung zur Begrenzung oder Reduzierung der Strahlungsintensität im Innenraum (2) umfasst.

3. Anordnung nach Anspruch 2,
   **dadurch gekennzeichnet, dass**
   die Vorrichtung zur Begrenzung oder Reduzierung der Strahlungsintensität die Ausbildung der zweiten Wand- und/oder Deckenflächen (4) aus Glas- und/oder Plexiglasscheiben mit veränderbarem Transmissionsvermögen umfasst.

4. Anordnung nach Anspruch 2 oder 3,
   **dadurch gekennzeichnet, dass**
   die Vorrichtung zur Begrenzung oder Reduzierung der Strahlungsintensität eine steuerbare Ein- und/oder Ausfahrung von Solarzellen umfasst.

5. Anordnung nach einem der Ansprüche 2 bis 4,
   **dadurch gekennzeichnet, dass**
   die Vorrichtung zur Begrenzung oder Reduzierung der Strahlungsintensität strahlungsabsorbierende Gegenstände umfasst, die im Innenraum (2) angeordnet sind.

6. Anordnung nach einem der Ansprüche 2 bis 5,
   **dadurch gekennzeichnet, dass**
   die Vorrichtung zur Begrenzung oder Reduzierung der Strahlungsintensität einen im Innenraum (2) angeordneten dunklen Stein mit einer spiegelnden Abdeckung, die öffenbar oder entfernbar ist, umfasst.

7. Anordnung nach einem der vorhergehenden Ansprüche,
   **dadurch gekennzeichnet, dass**
   die Vorrichtung zur Begrenzung oder Reduzierung der Strahlungsintensität zur Ausleitung der Strahlung vorgesehene, in den ersten Wand-und/oder Deckenflächen (3) vorgesehene oder im Boden (11) angeordnete Lichtleiter umfasst.

**8.** Anordnung nach einem der vorhergehenden Ansprüche,
**dadurch gekennzeichnet, dass**
die Sonnenlichtsauna oder das Gewächshaus eine Blitzschutzeinrichtung aufweist.

**9.** Anordnung nach einem der vorhergehenden Ansprüche,
**gekennzeichnet durch**
eine Konzentrieranordnung zur Konzentrierung von Sonnenlicht, mittels der für die Einleitung in die Sonnenlichtsauna oder das Gewächshaus bestimmtes Sonnenlicht konzentrierbar ist.

**10.** Anordnung nach Anspruch 9,
**dadurch gekennzeichnet, dass**
die Konzentrieranordnung derart ausgebildet ist, dass sie Sonnenlicht in einer Einfallrichtung stärker hindurch lässt als in einer Ausfallrichtung.

**11.** Anordnung nach Anspruch 10,
**dadurch gekennzeichnet, dass**
die Konzentrieranordnung ein Material umfasst, das Sonnenlicht in einer Einfallrichtung stärker hindurch lässt als in einer Ausfallrichtung.

**Claims**

**1.** Arrangement comprising

a) a sunlight sauna (1) or a greenhouse

a1) with wall and/or ceiling surfaces (3, 4) enclosing an internal space (2) and with a floor (11),
a2) wherein at least a first (3) of the wall and/or ceiling surfaces and/or the floor (11) is designed to be at least predominantly specularly and/or diffusely reflective for visible light and/or infra-red radiation and/or UV radiation towards the internal space,
a3) wherein at least a second (4) of the wall and/or ceiling surfaces is designed to be transparent, at least in sections, for visible light and/or infra-red radiation and/or UV radiation,
a4) wherein on and/or in the second wall and/or ceiling surfaces (4) one or more light funnels (5) are arranged or designed in such a way that a narrowest point (6) of the light funnel (5) is facing the internal space (2),
a5) wherein the peripheral surfaces (7) of the light funnels (5) are formed of specularly reflective side surfaces (8) of mirror elements neighbouring each other (12),
a6) wherein the mirror elements (12) have base surfaces (9) that face the internal space and between which the narrowest points (6) of the light funnels (5) are located,
a7) wherein the base surfaces (9) are designed to be at least predominantly specularly and/or diffusely reflective for visible light and/or infra-red radiation and/or UV radiation towards the internal space (2), and

b) at least one measuring device to determine the intensity of radiation in the internal space (2) of the sunlight sauna (1) or of the greenhouse, and
c) at least one display outside the sunlight sauna (1) or the greenhouse for the readings determined by the measuring device.

**2.** Arrangement according to claim 1,
**characterised in that**
the arrangement comprises at least one device to limit or reduce the intensity of radiation in the internal space (2).

**3.** Arrangement according to claim 2,
**characterised in that**
the device to limit or reduce the intensity of radiation comprises the construction of the second wall and/or ceiling surfaces (4) of panes of glass and/or panes of Plexiglas with variable transmittance.

**4.** Arrangement according to claim 2 or 3,
**characterised in that**

the device to limit or reduce the intensity of radiation comprises a controllable inward or outward movement of solar cells.

**5.** Arrangement according to one of the claims 2 to 4,
**characterised in that**
the device to limit or reduce the intensity of radiation comprises radiation-absorbing objects, which are located in the internal space (2).

**6.** Arrangement according to one of the claims 2 to 5,
**characterised in that**
the device to limit or reduce the intensity of radiation comprises a dark stone located in the internal space (2), which stone has a reflective cover that can be opened or removed.

**7.** Arrangement according to one of the preceding claims,
**characterised in that**
the device to limit or reduce the intensity of radiation comprises light guides provided to divert the radiation, provided in the first wall and/or ceiling surfaces (3) or located in the floor (11).

**8.** Arrangement according to one of the preceding claims,
**characterised in that**
the sunlight sauna or greenhouse is equipped with a lightning protection device.

**9.** Arrangement according to one of the preceding claims,
**characterised by**
a concentrating arrangement to concentrate sunlight, which enables sunlight that is to be conveyed into the sunlight sauna or the greenhouse to be concentrated.

**10.** Arrangement according to claim 9,
**characterised in that**
the concentrating arrangement is constructed in such a way that it allows sunlight through more strongly in an incident direction than in an exitant direction.

**11.** Arrangement according to claim 10,
**characterised in that**
the concentrating arrangement comprises a material which allows sunlight through more strongly in an incident direction than in an exitant direction.

**Revendications**

**1.** Agencement comprenant

a) un sauna solaire (1) ou une serre

a1) avec des faces de paroi et/ou de plafond (3, 4) entourant un espace intérieur (2) et un fond (11),
a2) où au moins une première (3) des faces de paroi et/ou de plafond et/ou le fond (11) est orienté vers l'espace intérieur au moins majoritairement et/ou est réalisée avec une réflexion diffuse de la lumière visible et/ou du rayonnement infrarouge et/ou du rayonnement UV,
a3) où au moins une deuxième (4) des faces de paroi et/ou de plafond est réalisée au moins par sections d'une manière transparente pour la lumière visible et/ou le rayonnement infrarouge et/ou le rayonnement UV,
a4) où sont disposés ou réalisés à et/ou dans la ou les deuxièmes faces de paroi et/ou de plafond (4) un ou plusieurs anamorphoseurs de lumière (5) de telle sorte qu'un emplacement le plus étroit (6) de l'anamorphoseur de lumière (5) soit orienté vers l'espace intérieur (2),
a5) où les faces de bord (7) des anamorphoseurs de lumière (5) sont formées par des faces latérales (8) orientées de manière réfléchissante de corps de miroir avoisinants (12),
a6) où les corps de miroir (12) présentent des faces de base (9) orientées vers l'espace intérieur, entre lesquelles sont réalisés les emplacements les plus étroits (6) des anamorphoseurs (5),
a7) où les faces de base (9) sont orientées vers l'espace intérieur (2) au moins majoritairement et/ou sont réalisées avec une réflexion diffuse pour la lumière visible et/ou le rayonnement infrarouge et/ou le rayonnement UV, et

b) au moins une installation de mesure pour la détermination de l'intensité du rayonnement dans l'espace intérieur (2) du sauna solaire (1) ou de la serre, et

c) au moins un affichage pour les valeurs de mesure déterminées par l'installation de mesure à l'extérieur du sauna solaire (1) ou de la serre.

**2.** Agencement selon la revendication 1, **caractérisé en ce que** l'agencement comprend au moins un dispositif pour la limitation ou la réduction de l'intensité du rayonnement dans l'espace intérieur (2).

**3.** Agencement selon la revendication 2, **caractérisé en ce que** le dispositif pour la limitation ou la réduction de l'intensité de rayonnement comprend la réalisation des deuxièmes faces de paroi et/ou de plafond (4) en plaques de verre et/ou plexiglas d'un pouvoir de transmission modifiable.

**4.** Agencement selon la revendication 2 ou 3, **caractérisé en ce que** le dispositif pour la limitation ou la réduction de l'intensité de rayonnement comprend une entrée et/ou sortie apte à être commandée de piles solaires.

**5.** Agencement selon l'une des revendications 2 à 4, **caractérisé en ce que** le dispositif pour la limitation ou la réduction de l'intensité de rayonnement comprend des objets absorbants le rayonnement, qui sont disposés dans l'espace intérieur (2).

**6.** Agencement selon l'une des revendications 2 à 5, **caractérisé en ce que** le dispositif pour la limitation ou la réduction de l'intensité de rayonnement comprend une pierre sombre disposée dans l'espace intérieur (2) avec un recouvrement réfléchissant qui peut être ouvert ou retiré.

**7.** Agencement selon l'une des revendications précédentes, **caractérisé en ce que** le dispositif pour la limitation ou la réduction de l'intensité de rayonnement comprend des conducteurs de lumière prévus pour l'évacuation du rayonnement, prévus dans les premières faces de paroi et/ou de plafond (3) ou disposés dans le sol (11).

**8.** Agencement selon l'une des revendications précédentes, **caractérisé en ce que** le sauna solaire ou la serre comporte une installation de protection contre la foudre.

**9.** Agencement selon l'une des revendications précédentes, **caractérisé par** un agencement de concentration pour la concentration de la lumière solaire au moyen duquel la lumière solaire destinée à l'introduction dans le sauna solaire ou dans la serre peut être concentrée.

**10.** Agencement selon la revendication 9, **caractérisé en ce que** l'agencement de concentration est réalisé de telle sorte qu'il laisse passer la lumière solaire dans une direction d'incidence plus fortement que dans une direction de sortie.

**11.** Agencement selon la revendication 10, **caractérisé en ce que** l'agencement de concentration comprend un matériau qui laisse passer la lumière solaire plus fortement dans une direction d'incidence que dans une direction de sortie.

**FIG 1**

**FIG 2**

**FIG 3**

**FIG 4**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19942632 A1
- US 1772219 A
- FR 2785792
- DE 4242258 A1
- US 4974922 A
- WO 9325174 A
- DE 20014497 U1
- DE 19937448 A1
- US 20010011551 A1
- US 4715358 A
- US 4003638 A
- EP 0114977 B1